# NEW EUROPEAN PATENT SPECIFICATION

(11) **EP 1 108 787 B2**
(45) Date of publication and mention of the opposition decision: **10.12.2008**
(45) Mention of the grant of the patent: 05.01.2005
(21) Application number: 00204190.3
(22) Date of filing: 24.11.2000
(51) Int. Cl.: C12N 15/34, C12N 5/10, C07K 14/11, C07K 14/075, C12N 15/85, C12N 7/02, A61K 39/145

(54) **Production of vaccines**
Vakzinproduktion
Production de vaccins

(30) Priority: 26.11.1999 EP 99203983
(43) Date of publication of application: 20.06.2001
(60) Divisional application: 04105656.5
(73) Proprietor: Crucell Holland B.V., 2333 CN Leiden (NL)
(72) Inventor: Pau, Maria Grazia, 2333 CW Leiden (NL); Uytdehaag, Alphonsus Gerardus Cornelius Maria, 3452 EH Vleuten (NL); Schouten, Govert Johan, 2353 EM Leiderdorp (NL)
(74) Representative: Jaenichen, Hans-Rainer

(56) References cited:
- WO-A-99/24068
- US-A- 5 192 539
- FALLAUX FJ ET AL: "New helper cells and matched early region 1-deleted adenovirus vectors prevent generation of replication-competent adenoviruses" HUMAN GENE THERAPY, vol. 9, no. 13, 1 September 1998 (1998-09-01), pages 1909-1917, XP002111070

## Description

### FIELD OF THE INVENTION

The invention relates to the development and manufacturing of vaccines. In particular the invention relates to the field of production of viral proteins and/or viruses, more in particular to the use of cells derived from a human embryonic retinoblast for the production of viruses growing in eukaryotic, preferably mammalian and in particular human cells. The invention is particularly useful for the production of vaccines to aid in protection against viral pathogens for vertebrates, in particular mammalians and especially humans. Thus the invention relates to the embodiments characterized in the claims.

Means and methods are disclosed herein for producing a virus and/or viral protein in a (human) cell, preferably using a defined synthetic medium, and for purifying the virus and/or components thereof from the cell and/or culture medium. Pharmaceutical compositions containing virus or its components and methods for manufacturing and recovering and/or purifying them are provided.

### BACKGROUND

Vaccination is the most important route of dealing with viral infections. Although a number of antiviral agents is available, typically these agents have limited efficacy. Administering antibodies against a virus may be a good way of dealing with viral infections once an individual is infected (passive immunisation) and typically human or humanised antibodies do seem promising for dealing with a number of viral infections, but the most efficacious and safe way of dealing with virus infection is and probably will be prophylaxis through active immunisations. Active immunisation is generally referred to as vaccination and vaccines comprise at least one antigenic determinant of typically a virus, preferably a number of different antigenic determinants of at least one virus or other pathogen, e.g. by incorporating in the vaccine at least one (viral) polypeptide or protein derived from a virus (subunit vaccines). Typically the formats mentioned sofar include adjuvants in order to enhance an immune response. This also is possible for vaccines based on whole virus (pathogen), e.g. in an inactivated format. A further possibility is the use of live, but attenuated forms of the pathogenic virus. A further possibility is the use of wild-type virus, e.g. in cases where adult individuals are not in danger from infection, but infants are and may be protected through maternal antibodies and the like.
Production of vaccines is not always an easy procedure. In some cases the production of viral material is on eggs, which leads to difficult to purify material and extensive safety measures against contamination, etc. Also production on bacteria and or yeasts, which sometimes but not always is an alternative for eggs requires many purification and safety steps. Production on mammalian cells would be an alternative, but mammalian cells used so far all require for instance the presence of serum and/or adherence to a solid support for growth. In the first case again purification and safety and e.g. the requirement of protease to support the replication of some viruses become an issue. In the second case high yields and ease of production become a further issue. The present invention overcomes at least a number of the problems encountered with the production systems for production of viruses and/or viral proteins for vaccine purposes of the systems of the prior art.

### DETAILED DESCRIPTION

The present invention discloses a novel human immortalized cell line for the purpose of propagating and harvesting virus, for production of said virus. PER.C6 cells (WO 97/00326) were generated by transfection of primary human embryonic retina cells, using a plasmid that contained the Ad serotype 5 (Ad5) E1A- and E1B-coding sequences (Ad5 nucleotides 459-3510) under the control of the human phosphoglycerate kinase (PGK) promoter.

The following features make PER.C6 or a derivative particularly useful as a host for virus production: it is a fully characterized human cell line, it was developed in compliance with GLP, it can be grown as suspension cultures in defined serum-free medium, devoid of any human or animal serum proteins; its growth is compatible with roller bottles, shaker flasks, spinner flasks and bioreactors, with doubling times of about 35 h.

### Influenza epidemiology.

Influenza viruses, members of the family of Orthomyxoviridae, are the causative agents of annual epidemics of acute respiratory disease. In the US alone 50 million Americans get flu each year. Estimated deaths worldwide (1972-1992) are 60.000 (CDC statistics). There have been 3 major cases of pandemics outbreaks of Influenza, namely in 1918 (Spanish flu, est. 40 million deaths), in 1957 (Asian flu, est. 1 million deaths), and in 1968 (Hong-Kong flu, est. 700.00 deaths). Infections with Influenza viruses are associated with a broad spectrum of illnesses and complications that result in substantial worldwide morbidity and mortality, especially in older people and patients with chronic illness. Vaccination against Influenza is most effective in preventing the often fatal complications associated with this infection (Murphy, B.R and R.G. Webster 1996). The production of Influenza virus on the diploid human cell line MRC-5 has been reported (Herrero-Euribe L et al 1983). However, the titers of Influenza virus are prohibitively low.

### Strains of Influenza virus

Present day flu vaccines contain purified Hemagglutinin and neuraminidase of Infuenza virus A and B. The 3 viruses that represent epidemiological important strains are Influenza A (H1N1), Influenza A (H3N2) and Influenza B. The division into A and B types is based on antigenic differences between their nucleoprotein (NP) and matrix (M) protein antigen. The Influenza A virus is further subdivided into subtypes based on the antigenic composition (sequence) of haemagglutinin (H1-H15) and neuraminidase (N1-N9) molecules. Representatives of each of these subtypes have been isolated from aquatic birds, which probable are the primordial reservoir of all Influenza viruses for avian and mammalian species. Transmission has been shown between pigs and humans and recently (H5N1) between birds and humans.

### Influenza vaccines

Three types of inactivated Influenza vaccine are currently used in the world: whole virus, split product and surface antigen or subunit vaccines. These vaccines all contain the surface glycoproteins, haemagglutinin (HA) and neuraminidase (NA), of the Influenza virus strains that are expected to circulate in the human population in the upcoming season. These strains, which are incorporated in the vaccine, are grown in embryonated hens' eggs, and the viral particles are subsequently purified before further processing.
The need for the yearly adjustment of Influenza vaccines is due to antigen variation caused by processes known as "Antigenic drift" and "Antigenic shift".

"Antigenic drift" occurs by the accumulation of a series of point mutations in either the H or N protein of a virus resulting in amino acid substitutions. These substitutions prevent the binding of neutralizing antibodies, induced by previous infection, and the new variant can infect the host.

"Antigenic shift" is the appearance of a new subtype by genetic reassortment between animal and human Influenza A viruses. The pandemic strains of 1957 (H2N2) and 1968 (H3N2) are examples of reassorted viruses by which avian H and or N genes were introduced in circulating human viruses, which subsequently could spread among the human population.

Based on the epidemiological surveys by over hundred National Influenza Centres worldwide, the World Health Organization (WHO) yearly recommends the composition of the Influenza vaccine, usually in February for the Northern hemisphere, and in September for the Southern hemisphere. This practice limits the time window for production and standardization of the vaccine to a maximum of 9 months. In case of an urgent demand of many doses of vaccine, for example when a novel subtype of Influenza A virus arises by Antigenic shift and Antigenic drift, limited availability of eggs may hamper the rapid production of vaccine. Further disadvantages of this production system are the lack of flexibility, the risk of the presence of toxins and the risks of adventitious viruses, particularly retroviruses, and concerns about sterility. This presents a serious problem in today's practice of Influenza vaccine production on embryonated hens' eggs.

Therefor, the use of a cell culture system for Influenza vaccine production would be an attractive alternative. Influenza viruses can be grown on a number of primary cells, including monkey kidney, calf kidney, hamster kidney and chicken kidney. Yet, their use for vaccine production is not practical, because of the need to re-establish cultures from these primary cells for each preparation of a vaccine. Therefor, the use of continuous immortalised cell lines for Influenza vaccine production is an attractive alternative.

The use of culture systems was facilitated by the realization that the proteolytic cleavage of HA in its two subunits (HA1 and HA2) is required for Influenza virus infectivity, and can be obtained by the addition of trypsin. Inclusion of trypsin permits replication and plaque formatior in Madin-Darby canine kidney (MDCK) cells (Tobita et al 1975).

The MDCK cell line was recently shown to support the growth of Influenza virus for vaccine production (Brand et al 1996 and 1997, Palache et al 1997). The use of trypsin requires growth of the MDCK cells in serum-free tissue culture medium (MDCK-SF1). However, MDCK cells are currently not approved as a substrate for production of Influenza virus.

Importantly, any non-human system for production of Influenza vaccines has an inherent drawback, known as 'adaptation'. Human Influenza A and B virus both carry mutations in the HA, due to adaptation in embryonated hens' eggs. These mutations result in altered antigenicity (Newman et al 1993, Williams and Robertson 1993, Robertson et al 1994, Gubareva et al 1994, Schild et al 1993, Robertson et al 1987, Kodihalli et al 1995). In humans, immunization with vaccines containing HA bearing an egg-adaptation mutation induces less neutralizing antibody to virus that contains a non-egg adapted HA (Newman et al 1993).

Human Influenza viruses propagated in canine cells such as MDCK cells also show adaptation, albeit to a lesser extent. Such viruses resemble the original human isolates more closely than egg derived viruses (Robertson et al 1990).

Furthermore there is evidence that host-specific changes in NA and host-specific phosphorylation patterns of NA can affect the replication of Influenza viruses (Schulman and Palese 1977; Sugiara and Ueda 1980; Kistner et al 1976).

Therefor, it would clearly be advantageous to avoid adaptation or other host-induced changes of Influenza virus. It may result in a more homogeneous population of viruses and render the ultimate vaccine more effective.

It is therefor an object of the present invention to provide human cells as a substrate for the production of high titers of Influenza virus, suitable for the development of vaccines.

### Rotavirus and rotavaccines

Rotaviruses are the most important cause of severe dehydrating gastroenteritis in young children worldwide. In developing countries infections with Rotaviruses lead to over 800.000 deaths annually. In the United States estimated costs of health care due to Rotavirus infections exceed 1 billion US dollars per year.

Rotaviruses, members of the family of Reoviridae, are double strand RNA viruses consisting of 11 RNA segments, each coding for a structural or non-structural viral protein (VP). This inner core of the virus comprises four VP's: VP1, 2, 3 and 6. The VP determine the three main antigenic properties of HRV-group, subgroup and serotype. Seven antigenically distinct groups (A-G) have been identified, that are encoded by the VP6. Infection with human Rotavirus (HRV) is predominantly caused by group A rotaviruses, with serotypes 1-4 accounting for 95% of clinical illness. Natural disease protection is serotype specific . Group A is further classified in subgroup I and II.

The double layer outer shell forming the viral capsid consists of two viral proteins, VP4 and VP7, that are the neutralization antigens involved in protective immunity and that determine the serotype, although the VP4 plays a minor role in serotype determination. During co-infection with different serotypes the segmented genomes readily undergo genetic reassorting, a property that has been used to create a vaccine (Marsha et al 1999).

Given the worldwide prevalence of rotavirus associated infant morbidity and mortality, large scale vaccination against Rotavirus is considered the most effective way to combat this virus. The goal of vaccination would not be to prevent the disease but to reduce its severity and complication, especially during the first few years of life.

The only effective vaccine available at present is a live attenuated orally delivered vaccine based on the reassortment of RNA segments of human rotaviruses, encoding the VP7's of serotypes 1,2 and 4 in a Rhesus rotavirus supplying the attenuated background together with the VP7 of serotype 3. Vaccination with this human rhesus reassortant tetravalent vaccine (RRV-TV), although highly effective in preventing severe gastroenteritis, is associated with intussuception, a bowel obstruction disease. For that reason this vaccine is no longer in use.

### DESCRIPTION OF THE INVENTION

The invention provides a method for producing a virus and/or viral proteins other than adenovirus or adenoviral proteins for use as a vaccine comprising providing a cell with at least a sequence encoding at least one gene product of the E1 gene or a functional derivative thereof of an adenovirus, providing said cell with a nucleic acid encoding said virus, culturing said cell in a suitable medium and allowing for propagation of said virus and harvesting said virus and/or viral proteins from said medium and/or said cell, wherein said cell is derived from a human embryonic retinoblast.

Until the present invention there are few, if any (human) cells that have been found suitable to produce viruses and/or viral proteins for use as vaccines in any reproducible and scaleable manner and/or sufficiently high yields and/or easily purifiable. We have now found that cells derived from a human embryonic retinoblast, which comprise adenoviral E1 sequences, preferably in their genome are capable of sustaining the propagation of viruses in significant amounts.

The cell according to the invention is derived from a human primary cell, preferably a cell which is immortalised by a gene product of said E1 gene. In order to be able to grow a primary cell it of course needs to be immortalized. The cell is one derived from a human embryonic retinoblast.

In cells according to the invention it is important that the E1 gene sequences are not lost during the cell cycle. It is therefore preferred that said sequence encoding at least one gene product of the E1 gene is present in the genome of said (human) cell. For reasons of safety care is best taken to avoid unnecessary adenoviral sequences in the cells according to the invention. It is thus another embodiment of the invention to provide cells that do not produce adenoviral structural proteins. However, in order to achieve large scale (continuous) virus production through cell culture it is preferred to have cells capable of growing without needing anchorage. The cells of the present invention have that capability. To have a clean and safe production system from which it is easy to recover and, if desirable, to purify the virus, it is preferred to have a method according to the invention, whereby said human cell comprises no other adenoviral sequences. The most preferred cell for the methods and uses of the invention is PER.C6 as deposited under ECACC no. 96022940, or a derivative thereof.

Thus the invention provides a method using a cell according to the invention, wherein said cell further comprises a sequence encoding E2A or a functional derivative or analogue or fragment thereof, preferably a cell wherein said sequence encoding E2A or a functional derivative or analogue or fragment thereof is present in the genome of said human cell and most preferably a cell wherein said E2A encoding sequence encodes a temperature sensitive mutant E2A.

Furthermore, as stated the invention also provides a method according to the invention wherein said (human) cell is capable of growing in suspension.

The invention also provides a method wherein said human cell can be cultured in the absence of serum. The cells according to the invention, in particular PER.C6 has the additional advantage that it can be cultured in the absence of serum or serum components. Thus isolation is easy, safety is enhanced and reliability of the system is good (synthetic media are the best in reproducibility). The human cells of the invention are capable of normal post and peritranslational modifications and assembly. This means that they are very suitable for preparing viral proteins and viruses for use in vaccines.

Thus the invention provides a method according to the invention, wherein said virus and/or said viral proteins comprise a protein that undergoes post-translational and/or peritranslational modification, especially wherein said modifications comprise glycosylation. A good example of a viral vaccine that has been cumbersome to produce in any reliable manner is Influenza vaccine. The invention provides a method wherein said viral proteins comprise at least one of an Influenza virus neuraminidase and/or a haemagglutinin. Other viral proteins (subunits) and viruses (wild-type to be inactivated) or attenuated viruses that can be produced in the methods according to the invention include enterovirus, such as rhinovirus, aphtovirus, or poliomyelitisvirus, herpesvirus, such as herpes symplex virus, pseudorabies virus or bovine herpes virus, orthomyxovirus, such as Influenza virus, a paramyxovirus, such as newcastle disease virus, respiratory syncitio virus, mumps virus or a measles virus, retrovirus, such as human immunedeficiency virus or a parvovirus or a papovavirus, rotavirus or a coronavirus, such as transmissable gastroenteritisvirus or a flavivirus, such as tick-borne encephalitis virus or yellow fever virus, a togavirus, such as rubella virus or eastern-, western-, or venezuelean equine encephalomyelitis virus, a hepatitis causing virus, such as hepatitis A or hepatitis B virus, a pestivirus, such as hog cholera virus or a rhabdovirus, such as rabies virus, a Bunyaviridae virus, such as Hantavirus.

In one embodiment a cell of the invention is useful in the generation of an influenza virus strain that does not grow very efficiently on embryonal eggs.

The invention also provides the use of a human cell having a sequence encoding at least one E1 protein of an adenovirus or a functional derivative, homologue or fragment thereof in its genome which cell does not produce structural adenoviral proteins for the production of a non-adenoviral virus for use in a vaccine, wherein said human cell is derived from a human embryonic retinoblast. Of course for such a use the cells preferred in the methods according to the invention are also preferred. The invention also provides the products resulting from the methods and uses according to the invention, especially viral proteins and viruses obtainable according to those uses and/or methods, especially when brought in a pharmaceutical composition comprising suitable excipients and in some formats (inactivated viruses, subunits) adjuvants. Dosage and ways of administration can be sorted out through normal clinical testing in as far as they are not yet available through the already registered vaccines.

Thus the invention also provides a virus or a viral protein for use in a vaccine obtainable by a method or by a use according to the invention, said virus or said viral being free of any non-human mammalian proteinaceous material and a pharmaceutical formulation comprising such a virus and/or viral protein.

The invention further provides a human cell having a sequence encoding at least one E1 protein of an adenovirus or a functional derivative, homologue or fragment thereof in its genome, which cell does not produce structural adenoviral proteins and having a nucleic acid encoding a non-adenoviral virus. This cell is derived from a human embryonic retinoblast and can be used in a method according to the invention.
In a preferred embodiment the invention provides Influenza virus obtainable by a method according to the invention or by a use according to the invention. In another embodiment the invention provides Influenza vaccines obtainable by a method according to the invention or by a use according to the invention.

Influenca virus preparations harvested from embryonal eggs typically need to be purified for the preparation of a vaccine. Purification typically entails at least one concentration step of the virus. Current technologies for the concentration of influenza virus from such relatively crude preparations of influenza virus are cumbersome.

A derivative of an infectious influenze virus of the invention typically is a virus, virus particle, or viral protein or part thereof that can be used for immunization purposes. Typically this entails a virus infectivity inactivation step.

### EXAMPLES

To illustrate the invention, the following examples are provided, not intended to limit the scope of the invention.

### Example 1

### MATERIALS AND METHODS

### PER.C6 and MDCK Cell culture

Madin Darby Canine Kidney (MDCK) cells were cultured in Dulbecco's modified Eagle's medium (DMEM, Life Technologies Breda, The Netherlands) containing 10% heat inactivated fetal bovine serum and lx L-Glutamin (Gibco-BRL), at 37°C and 10% CO₂. Suspension cultures of PER.C6 were cultured in ExCell 525 (JRH Biosciences) supplemented with lx L-Glutamin, at 37°C and 10% CO₂, in stationary cultures in 6 well dishes (Greiner) or in 490 cm² tissue culture roller bottles (Corning Costar Corporation) during continuous rotation at 1 rpm.

### Immunofluorescence test

Direct immunofluorescence assays for the detection of Influenza virus infection were carried out using the IMAGEN^{™} Influenza Virus A and B kit (Dako) according to the standard protocol of the supplier. Samples were viewed microscopically using epifluorescence illumination. Infected cells were characterised by a bright apple-green fluorescence.

### Propidium Iodide staining

Cell pellets were resuspended in 300 µl of cold PBS/0.5% BSA + 5 µl of propidium iodide (concentration 50 µg/ml) in PBS/FCS/azide solution known to persons skilled in the art. Viable and dead cells were then detected via flow cytofluorometric analysis.

### Haemagglutination assay

In general, haemagglutination assays for Influenza virus titers were performed according to methods known to persons skilled in the art. Here, 50 µl of a two-fold diluted virus solution in PBS was added to 25 µl PBS and 25 µl of a 1% suspension of turkey erythrocytes (Biotrading Benelux B.V.) in PBS and incubated in 96 well microtiter plates at 4°C for 1 h. The haemagglutination pattern was examined and scored, and expressed as hemagglutinating units (HAU's). The number of HAU's corresponded to the reciprocal value of the highest virus dilution that showed complete haemagglutination.

### Western blot analysis of the Influenza HA protein.

In general, obtained Influenza viruses were disrupted in a Laemmli buffer according to methods known to persons skilled in the art and different volumes of obtained protein mixtures were separated using 10% SDS/PAGE gels. In brief, blots were blocked for 30 min at room temperature with block solution (5% non fat dry milkpowder (Biorad) in TBST suplemented with 1% rabbitserum (Rockland)), followed by 3 washes with TBST. Then, the blots were incubated with the anti A/Sydney/5/97 HA antiserum (98/768 NIBSC) diluted 1/500 in 1%BSA/TBST with 5% rabbit serum (Rockland) O/N at room temperature. Again the blots were washed 8 times with TBST. Finally the blots were incubated with the rabbit anti sheep antiserum (HRP labelled, Rockland) 1/6000 diluted in block solution for 1 h at room temperature. After 8 washes with TBST the protein-conjugate complex was visualised with ECL (Amersham Pharmacia Biotech), and films (Hyperfilm, Amersham Life Science) were exposed. The antisera were obtained from the NIBSC (UK) and applied in dilutions recommended by the NIBSC.

### Single Radial Immunodiffusion (SRID) assay

The concentration of haemagglutinin in supernatants, derived from Influenza virus infected-PER.C6 cells, was determined by the single radial immunodiffusion (SRID) test as previously described (Wood et al 1977). The assay was performed using standard NIBSC (UK) antigens and antisera reagents.

### Plaque assay

A total of 1 ml of 10-fold serially diluted viral supernatants were inoculated on MDCK cells which were grown until 95% confluence in 6-well plates. After 1 h at 35°C the cells were washed twice with PBS and overloaded with 3 ml of agarose mix (1.2 ml 2.5% agarose, 1.5 ml 2x MEM, 30 µl 200 mM L-Glutamine, 24 µl trypsin-EDTA, 250 µl PBS). The cells were then incubated in a humid, 10% CO₂ atmosphere at 35°C for approximately 3 days and viral plaques were visually scored.

### Virus infectivity assay (TCID₅₀)

Titration of infectious virus was performed on MDCK cells. In brief, cells were seeded in 96 well plates at a density of 4x10⁴ cells/well in DMEM supplemented with 2mM L-Glutamin. Twenty-four hours later cells were infected with 100 µl of ten fold serially diluted culture supernatants, in quadruplicate, in medium containing Trypsin-EDTA at the concentration of 4 µg/ml. Two hours after infection cell monolayers were washed two times in PBS and incubated in medium containing trypsin for 7 days, at 35°C. Supernatants from these cultures were then tested in an HA assay. TCID₅₀ titers were calculated according to the method of Karber (1931).

### β-propiolactone Influenza virus inactivation

For inactivation of the viruses to obtain whole-inactivated virus for the generation of vaccines derived from PER.C6, a mutation protocol known to persons skilled in the art was performed using β-propiolactone. β-propiolactone is a very effective agent widely used for the inactivation of viruses and well known in the art for its mutating effects. It modifies nucleic acid bases of the viral genome and the host cell genome and blocks replication thereafter. Following an established protocol used to prepare the whole inactivated Influenza vaccine prepared on embryonated eggs, the amount of virus corresponding to approximately 400 µg of HA per strain was inactivated and used for the final vaccine formulation. Briefly, one volume of 0.3 M Sodium phosphate buffer was added to 9 volumes of Influenza virus preparation.
Inactivation of the viruses was carried out by adding one volume of 10% of β-propiolactone (Newall Design, UK) to 100 volumes of phosphate buffered virus preparation and incubated at 20°C for 24 h. Inactivation of the viruses was checked by plaque assay and no plaques were detected for any of the inactivated batches (data not shown).

### Example 2A

### PER.C6 Cell banking and preculture

Cell line PER.C6 (deposited under No. 96022940 at the European Collection of Animal Cell Cultures at the Centre for Applied Microbiology and Research), or derivatives thereof were used (described in WO 97/00326). Cell lines were banked by a two tier cell bank system. The selected cell line was banked in a research master cell bank (rMCB) which was stored in different locations. From this rMCB research working cell banks (rWCB) were prepared as follows: an ampoule of the rMCB was thawed, and cells were propagated until enough cells are present to freeze the cells by using dry ice. Up to 500 ampoules containing 1 ml (1-2x10⁶ cells/ml) of rWCB were stored in the vapour phase of a liquid N₂ freezer.
One ampoule containing 5x10⁶ PER.C6 cells of the WCB was thawed in a water bath at 37°C. Cells were rapidly transferred into a 50 ml tube and resuspended by adding 9 ml of the suspension medium ExCell 525 (JRH Biosciences) supplemented with 1 x L-Glutamin. After 3 min of centrifugation at 1000 rpm in atable top centrifuge, cells were resuspended in a final concentration of 3x10⁵ cells/ml and cultured in a T80 tissue culture flask, at 37°C, 10% CO₂. Two to three days later, cells were seeded into 490 cm² tissue culture roller bottles (Corning Costar Corporation), with a density of 3x10⁵ per ml and cultured in continuous rotation at 1 rpm.

### Example 2B

### PER.C6 cells as permissive cell line for Influenza A virus

PER.C6 as a human cell was not known for its ability to sustain Influenza virus infection and replication. It was therefore verified whether PER.C6 cells are permissive for Influenza virus infection in comparison with the dog cell line Madin Darby Canine Kidney (MDCK), that served as a positive control.

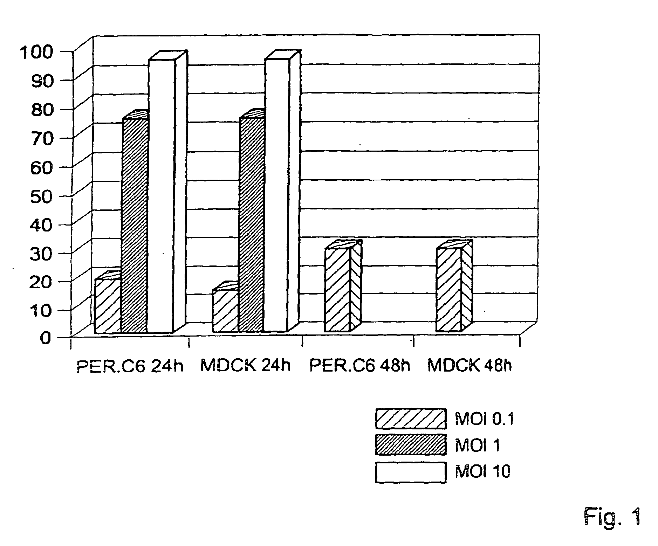

On the day before infection, 2x10⁵ MDCK cells per well were seeded in 6-well plates. Twenty four hours later, 4x10⁵ seeded PER.C6 and MDCK cells per well were infected with the H1N1 strain A/Puerto Rico/8/34 (titer 3.6x10⁷ pfu/ml), (obtained from Dr. E. Claas, Leiden University Medical Center, The Netherlands). Infection was performed at various multipliticies of infection (moi's) ranging from of 0.1 to 10 pfu/cell. After about 2 hours of incubation at 37°C, the inoculum was removed and replaced by fresh culture medium. A direct immunofluorescence assay for the detection of Influenza virus infection was performed 24 and 48 h post infection. The experiment showed permissiveness of PER.C6 for Influenza infection, with percentages of positive cells moi-dependent and comparable with MDCK (Fig.1).

### Example 3

### PER.C6 used for Influenza A virus propagation.

It was verified whether replication and propagation of Influenza virus could be supported by PER.C6. On the day of infection, PER.C6 cells were seeded in 490 cm² tissue culture roller bottles, with the density of 2x10⁵ cells/ml in a final volume of 40 ml, in the presence of 5 µg/ml of trypsine-EDTA (Gibco-BRL). Cells were either mock inoculated or infected with the H3N2 strain A/Shenzhen/227/95 (titer 1.5x10⁶ pfu/ml) (obtained from Dr. E. Claas, Leiden University Medical Centre, The Netherlands). Infections were performed at moi 10⁻⁴ and 10⁻³ pfu/cell. After 1 h of incubation at 37°C, the inoculum was removed by spinning down the cells at 1500 rpm and resuspending the cells in fresh culture medium + 5 µg/ml of trypsine-EDTA. Harvest of 1.3 ml of cell suspension was carried out each day, from day 1 to day 6 post-infection. Supernatants were stored at -80°C and used for haemagglutination assays. Cell pellets were used for direct immunofluorescence tests and for propidium iodide staining.

### Example 4

### Permissiveness of PER.C6 for different Influenza strains

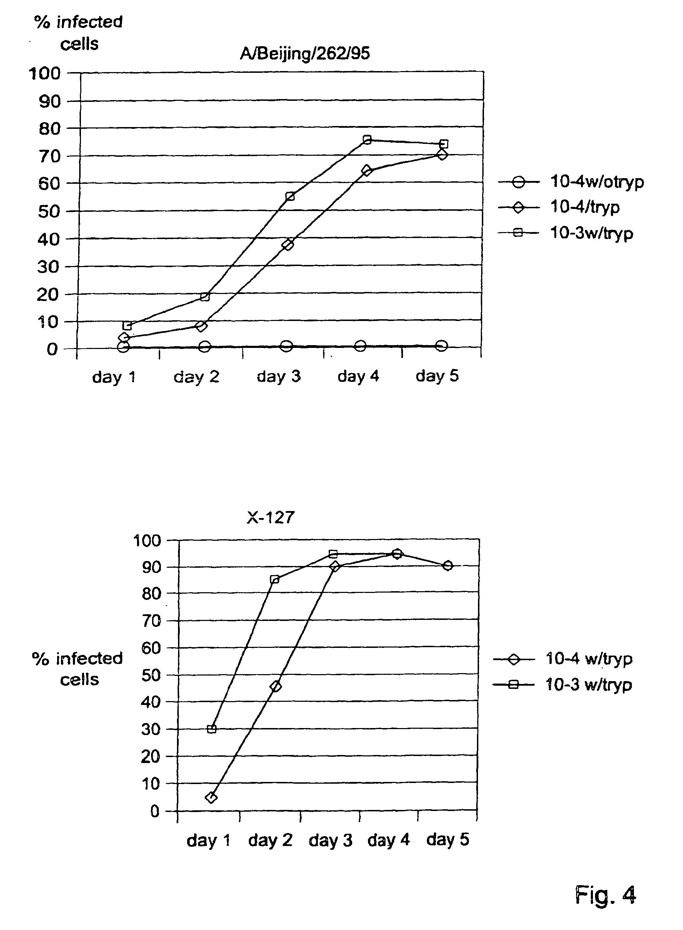

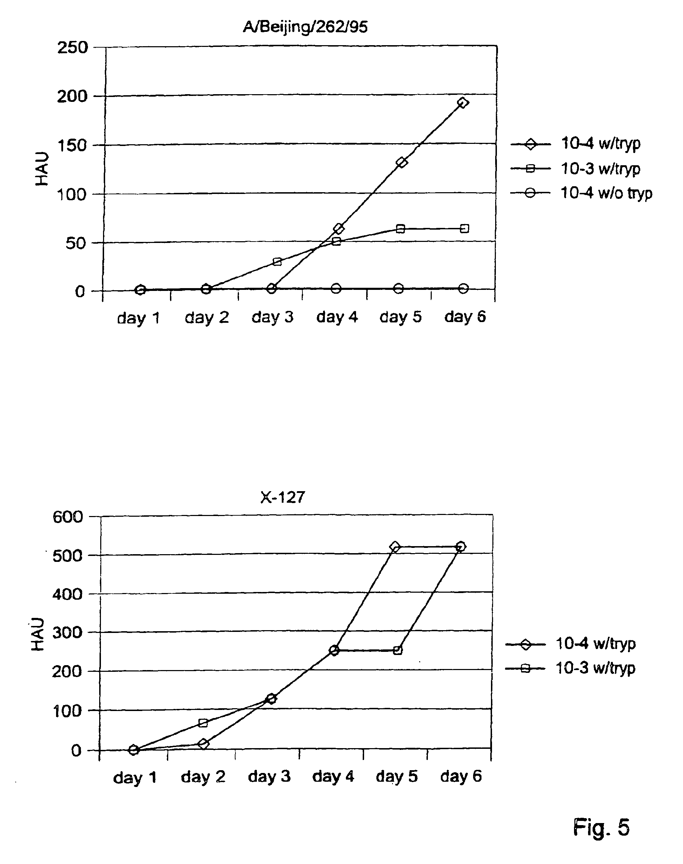

To further investigate the permissiveness of PER.C6 for propagation of various Influenza strains, an infection by using the H1N1 vaccine strains A/Beijing/262/95 and its reassortant X-127, obtained from the National Institute for Biological Standards and Control (NIBSC, UK) was performed. On the day of infection, PER.C6 cells were seeded in 490 cm² tissue culture roller bottles, with the density of approximately 1x10⁶ cells/ml in a final volume of 50ml. Cells were inoculated with 5µl (10⁻⁴ dilution) and 50 µl (10⁻³ dilut ion) of virus in the presence of 5 µg/ml trypsin-EDTA. In order to establish if trypsin was indeed required, one more infection was carried out by inoculating 5µl of the strain A/Beijing/262/95 in the absence of the protease. After approximately 1 h of incubation at 37°C, the inoculum was removed by spinning down the cells at 1500 rpm and resuspending them in fresh culture medium ± 5 µg/ml of trypsin-EDTA. At day 2 and day 4 post-infection more trypsin was added to the samples. Harvest of 1.3 ml of cell suspension was carried out from day 1 to day 6 post-infection. Supernatants were stored at -80°C and used for haemagglutination assays and further infections; cell pellets were used for direct immunofluorescence tests. Results obtained with the above mentioned immunofluorescence and haemagglutination assays are shown in Fig.4 and Fig.5, respectively, illustrating the efficient replication and release of the viruses.

### Example 5

### Infectivity of virus propagated on PER.C6

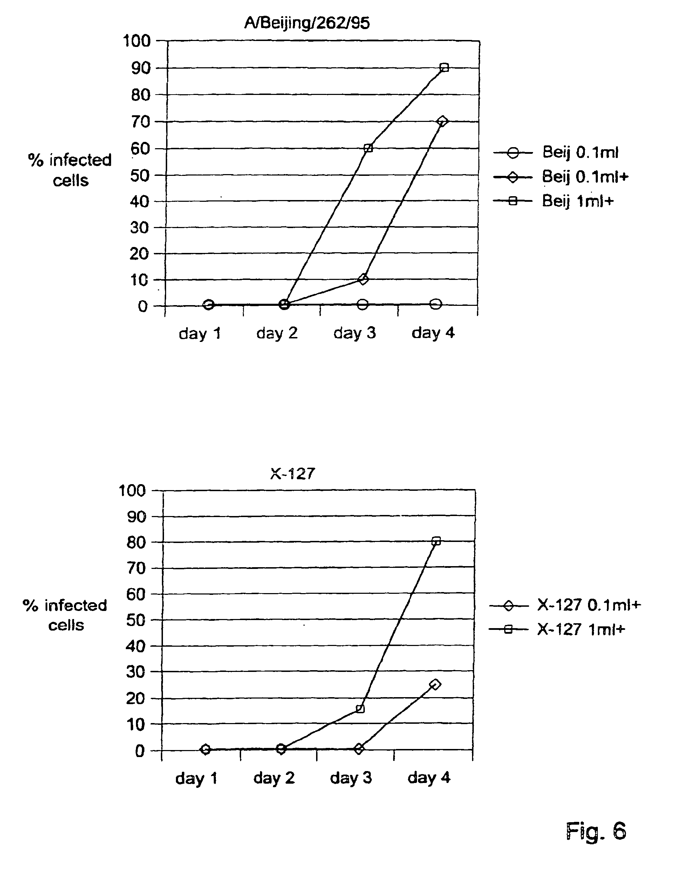

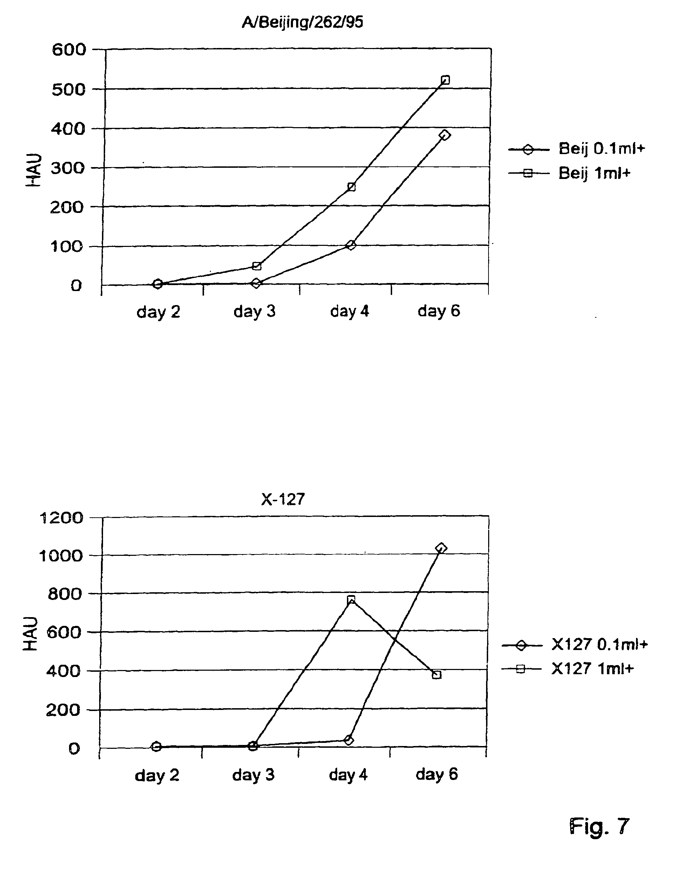

It was verified whether the viruses grown in PER.C6 were infectious and if adaptation to the cell line could increase virus yields. Virus supernatants derived from PER.C6 infected with the strains A/Beijing/262/95 and its reassortanc X-127 (dil.10-3) and harvested at day 6 post-infection, were used. At the day of infection, PER.C6 were seeded in 490 cm² tissue culture roller bottles, with the density of approximately 1x10⁶ cells/ml in a final volume of 50 ml. Cells were inoculated with 100 µl and 1 ml of virus supernatant in the presence of 5 µg/ml trypsin-EDTA. In order to establish if trypsin was still required, one more infection was carried out by inoculating 100 µl of the strain A/Beijing/262/95 in the absence of the protease. After approximately 1 hour of incubation at 37°C, the inoculum was removed by spinning down the cells at 1500 rpm and resuspending them in fresh culture medium ± 5 µg/ml of trypsin-EDTA. At day 2 and day 4 post-infection more trypsin was added to the samples. Harvest of 1.3 ml of cell suspension was carried out from day 1 to day 6 post-infection. Supernatants were stored at -80°C and used for haemagglutination assays and further infections; cell pellets were used for direct immunofluorescence tests.
Results obtained with the above mentioned immunofluorescence and haemagglutination assays are shown in Fig.6 and Fig.7. Data obtained with the present experiment showed infectivity of the viruses grown in PER.C6 as well as an increase in virus yields.

### Example 6

### The presence of cell surface receptors for Influenza virus on PER.C6.

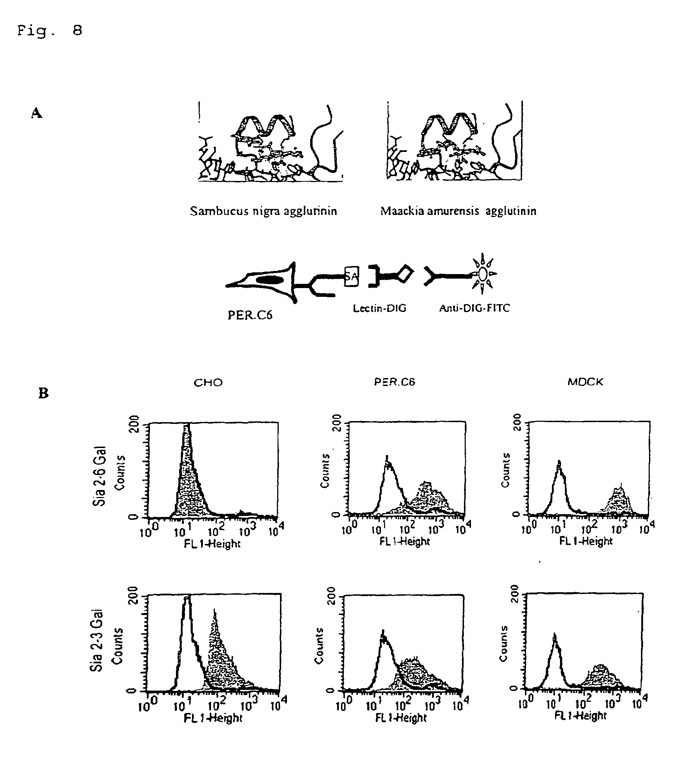

Propagation of human Influenza A and B strains in embryonated chicken eggs always leads to a selection of receptor-binding variants that harbor amino acid substitutions at the distal portion of the HA globular head in the exposed and functionally important regions of the molecule. Because of these mutations, the egg-adapted strains can differ from the original human viruses in their antigenic and immunogenic activities, as well as their virulence. Human Influenza viruses isolated from MDCK cells usually present a HA protein that is identical to the HA protein present on the virus of the original clinical sample. A recent study (Govorkova 1999) clarified the molecular basis for the selection of variants in chicken eggs and the absence of this variant selection phenomenon in MDCK cells. All human Influenza A and B strains isolated from MDCK cells were found to bind with high affinity and specificity for alpha2,6 sialic acid-galactose linkages present in oligosaccharides present in cell surface receptors, whereas their egg-grown counterparts showed an increased affinity for the alpha2,3 sialic acid-galactose linkages in cell surface receptors carrying oligosaccharides (Sia2-3Gal). Using specific lectins it was demonstrated that only Sia2-3Gal-containing receptors were present on the surface of chicken embryonic cells, whereas MDCK cells expressed both Sia2-6Gal and Sia2-3Gal. The expression of the Sia2-3Gal and Sia2-6Gal moieties on the surface of PER.C6 cells were studied by FACS analysis, using two different digoxigenin (DIG)-labeled lectins: Sambuca nigra agglutinin (SNA) that specifically recognises Sia2-6Gal linkages and the Maackia amurensis agglutinin (MAA), that specifically recognises Sia2-3Gal linkages. Fig.8A shows the recognition of the SNA and MAA lectins and their binding to the glycosylation sites. Furthermore, Fig.8A shows the schematic interaction between the FITC labelled anti-DIG antibody and the DIG-labelled lectin that recognises the specific sialyl bond in the glycosylation backbone of the receptor present on the cell surface. Both lectins were taken from the glycan differentiation kit (Boehringer-La Roche).

The experiment was carried out on PER.C6 cells in suspension and adherent MDCK and CHO cells. MDCK and CHO cells were released from the solid support using trypsin-EDTA (Gibco-BRL). The cell suspensions were then washed once with Mem-5% FBS and incubated in this medium for 1 hour at 37°C. After washing with PBS (Gibco-BRL), the cells were resuspended to a concentration of approximately 10⁶cells/ml in binding medium (Tris-buffered saline, pH 7.5, 0.5%BSA, and 1 mM each of MgCl₂, MnCl₂ and CaCl₂). Cell Aliquots were incubated for 1 h at room temperature with the DIG-labeled lectins SNA and MAA. After 1 h, lectin-treated cells were washed with PBS and incubated for an additional hour at room temperature with FITC-labeled anti-DIG antibody (Boehringer-Mannheim). Finally, the cells were washed with PBS and analysed by fluorescence activated cell sorting using a FAC-sort apparatus (Becton Dickinson). The results shown in Fig.8B demonstrate that PER.C6 cells were stained by both lectins showing the presence of the Sia2-6Gal as well as the Sia2-3Gal receptors.

In the same experiment MDCK cells were used as positive control for both the sialylated receptors, whereas CHO cells, due to the absence of the alpha 2-6 sialyltransferase glycosylation enzyme in these hamster cells, represented a negative control for the Sia2-6Gal moiety. The upper panels show results with the SNA lectin and the lower panels showing results with the MAA lectin. From these results it can be concluded that PER.C6 expresses cell surface proteins that have both Sia2-3Gal and Sia2-6Gal linkages in their oligosaccharide chains.

### Example 7

### Effect of different concentrations of trypsin-EDTA on the viability of PER.C6 cells, on the Influenza virus production in PER.C6 cells and on the HA protein derived thereof.

Due to the absolute trypsin requirement for the propagation of Influenza viruses in cell cultures, the effects of different concentrations of trypsin-EDTA on PER.C6 cell viability and virus replication in PER.C6 cells after infection using several Influenza strains were investigated.

### Infection with Influenza virus strain A/Sydney/5/97 in the presence of low concentrations of trypsin

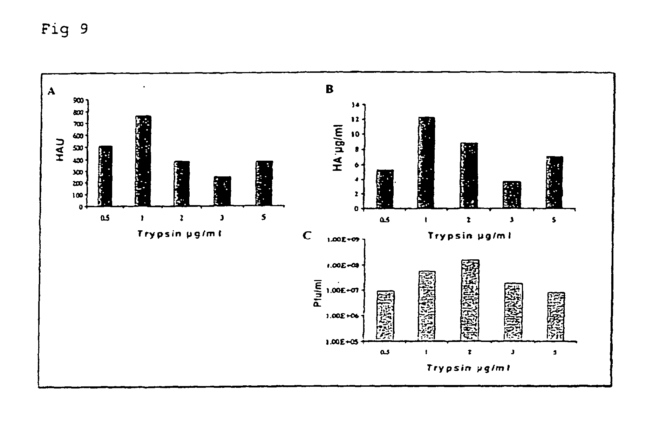

On the day of infection, PER.C6 cells were seeded in 490 cm² tissue culture roller bottles, at a density of 1x10⁶ cells/ml, in the presence of trypsin-EDTA at final concentrations of 0.5, 1, 2, 3 and 5 µg/ml.
These trypsin concentrations did not interfere with the growth characteristics of the cells and their viability (data not shown). Cells were either, mock infected or infected with PER.C6-grown Influenza virus A/Sydney/5/97 at an moi of 10⁻⁴ pfu/cell. The viral production was monitored by direct immunofluorescence (data not shown), haemagglutination assays, single-radial-immunodiffusion (SRID) above and plaque assays, all as described above. Results from this experiment are depicted in Fig.9 and show that the HA content as measured by SRID as well as the biological activity of the virus, expressed in HAU, were highest when a trypsin concentration of 1 µg/ml was used. Fig.9 also shows that by using a plaque assay the highest number of plaque forming units (pfu) per ml was observed in the sample corresponding to cells grown in medium containing 2 µg/ml of trypsin.

### Infection with Influenza virus strain B/Harbin/7/94.

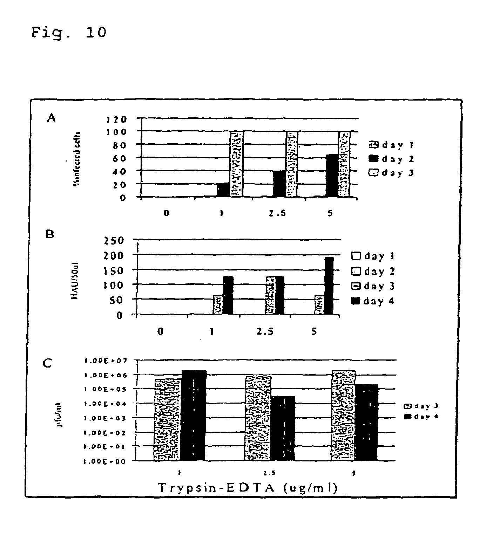

On the day of infection PER.C6 cells were seeded in 490 cm² tissue culture roller bottles at a density of 1x10⁶ cells/ml, in the presence of different concentrations of trypsin-EDTA, ranging from 1 to 5 µg/ml. Cells were infected with PER.C6-grown virus B/Harbin/7/94 at an moi of 10⁻³ pfu/cell. Production of the virus was monitored by direct immunofluorescence, haemagglutination and plaque assays as shown in Fig.10. The infectability of PER.C6 at day 2 increased with the concentration of trypsin. At day 3 however, no significant difference was observed in the percentage of infected cells when 1, 2.5 or 5 µg/ml trypsin was present. In the absence of trypsin (0 µg/ml) no Influenza virus infection was detected. At the day of the last harvest (day 4 post-infection), the biological activity of the virus, as measured by haemagglutination assay, did not differ significantly. Interestingly, the infectivity assay performed in samples that were taken at day 3 and 4 after infection, showed a difference in the production of the virus. The highest titers were obtained at day 3 and day 4 when a trypsin concentration of 2.5 to 5 (day 3) and 1 µg/ml (day 4) were used.

### Infection with Influenza virus reassortant X-127.

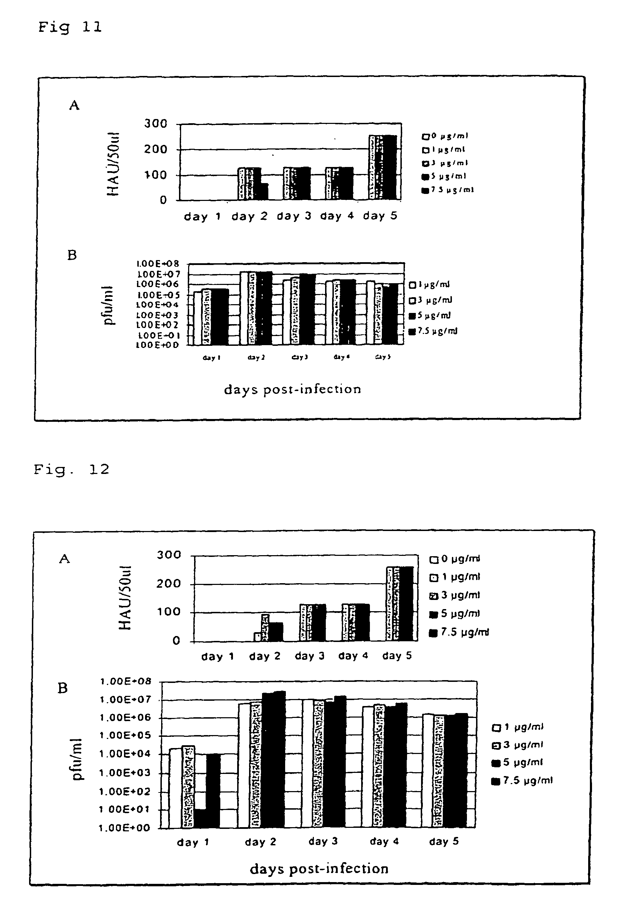

On the day of infection, PER.C6 cells were seeded in T25 tissue culture flasks, at a density of 1x10⁶ cells/ml, in the presence of different concentrations of trypsin-EDTA ranging from 0 to 7.5 µg/ml. Cells were infected with PER.C6-grown virus X-127 (egg-reassortant for the strain A/Beijing/262/95) at an moi of 10⁻⁴ and 10⁻³ pfu/cell. Viral growth was monitored by direct immunofluorescence, haemagglutination and plaque assays. As shown in Fig.11 and Fig.12, HAU titers were identical between samples, independent of the trypsin concentration and the initial moi that was used. Furthermore, no significant differences were observed in the infectivity titers, as measured by plaque assay.

### Infection of PER.C6 with Influenza virus strain A/Sydney/5/97 in the presence of high concentrations of trypsin

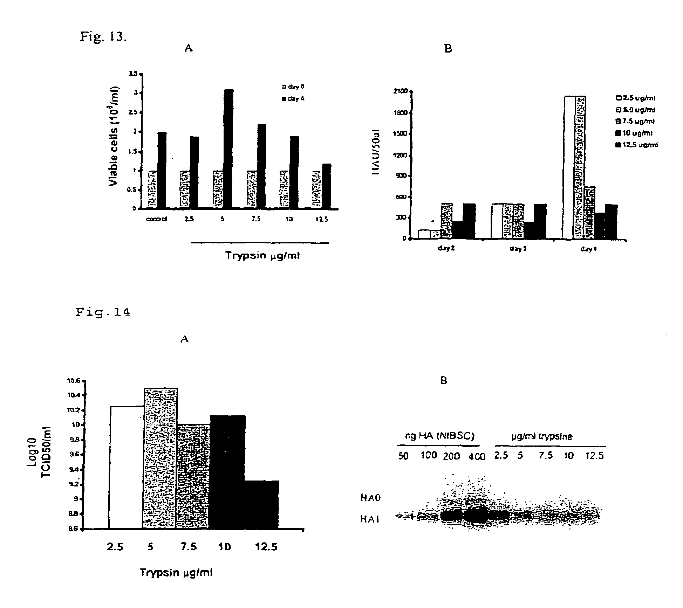

To test the effect of increasing concentrations of trypsin on viability of the cells and virus replication, PER.C6 cells were seeded in roller bottles at a density of 1x10⁶ cells/ml in the presence of various concentrations of trypsin-EDTA ranging from 0 to 12.5 ug/ml. Cells were either mock infected or infected with PER.C6 grown virus A/Sydney/5/97 virus at an moi of 4x10⁻⁵ pfu/cell. HAU's present in the obtained batches were determined as described. Importantly, data depicted in Fig.13 clearly show chat trypsin concentrations up to 10 µg/ml do not interfere with the cell viability. Moreover, the biological activity of the virus obtained at day 4 after infection as measured by HAU was higher when a trypsin concentration of 2.5 to 5 µg/ml was used. Furthermore, the TCID₅₀ was measured (Fig.14A) and plaque assays were performed (data not shown). No relevant differences were found in these plaque assays, in the infectivity titers (TCID₅₀), in the HA cleavage and quantity (approximately 10 µg/ml) as determined by western blot analysis shown in Fig.14B.

### Example 8

### Influenza virus production on PER.C6 cells in a hollow fiber-perfusion bioreactor system.

The scalability of Influenza virus production in suspension growing PER.C6 cells was studied by using 3 liter (total volume) bioreactors containing a 2 liter cell cell suspension volume in serum free medium, which is also free of animal or human derived proteins (ExCell 525, JRH Biosciences) .

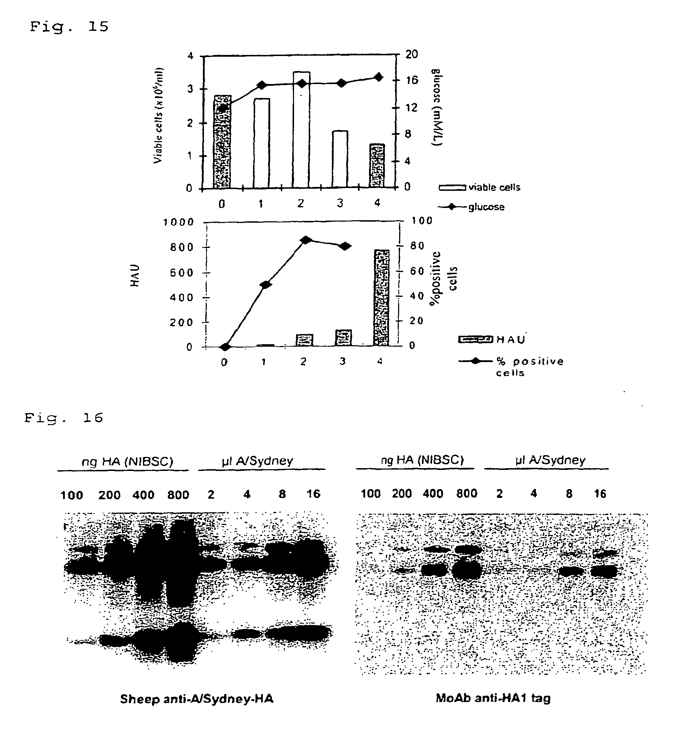

Influenza infection was carried out at a cell density of approximately 3x10⁶ cells/ml. Cells were inoculated with PER.C6-grown A/Sydney/5/97 virus, at an moi of 10⁻⁴ pfu/cell. Samples of 5 to 10 ml of cell suspensions were taken every day to perform general cell counts, to determine the viability of the cells, for glucose concentration measurements, for direct immunofluorescence, for haemagglutination and for infectivity assays. The results of these experiments are shown in Fig.15.

To investigate the presence and the status of the HA protein western blots using two different anti-HA antibodies obtained from NIBSC were used. SRID assays as described above were also performed. The results depicted in the two western blots in Fig.16 show that the Influenza virus batch produced in this bioreactor yielded an HA content of an estimated concentration of 15 µg/ml which was confirmed by SRID assays. The HA produced is comparable to reference NIBSC HA in terms of subunit composition and immune reactivity with the reference subtype specific antisera.

### Example 9

### Infection of PER.C6 with A/Sydney/5/97 in a 15 liter bioreactor followed by a specific Down Stream Process (DSP).

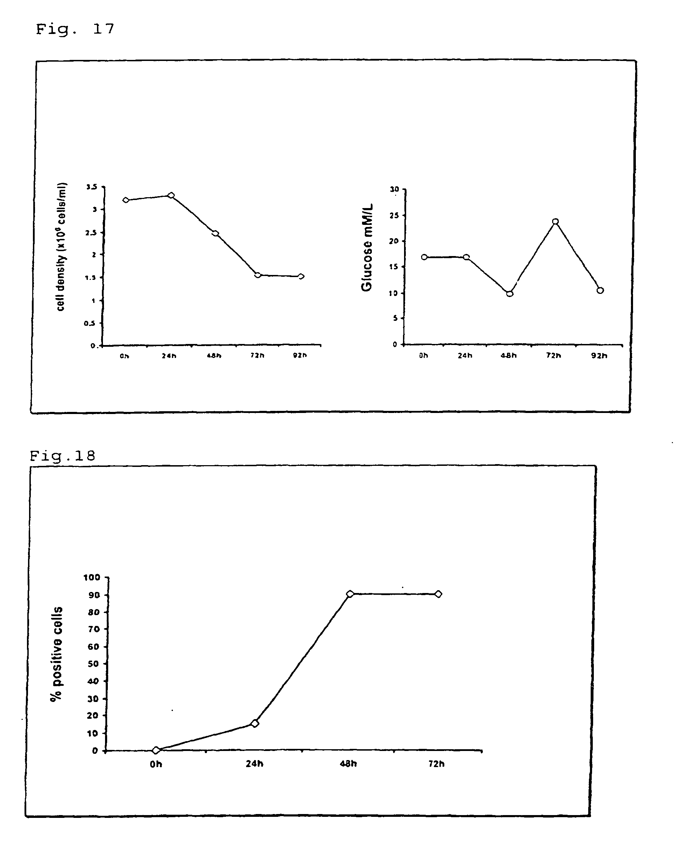

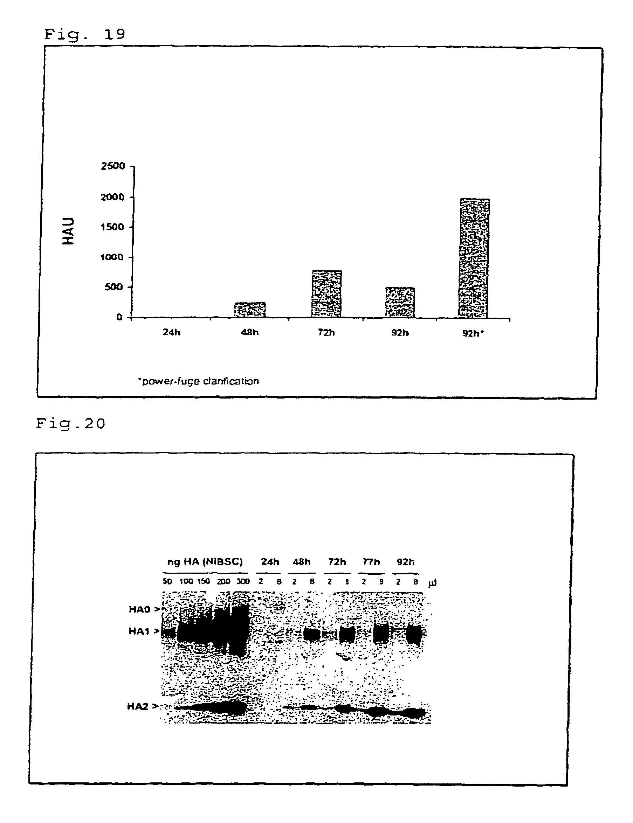

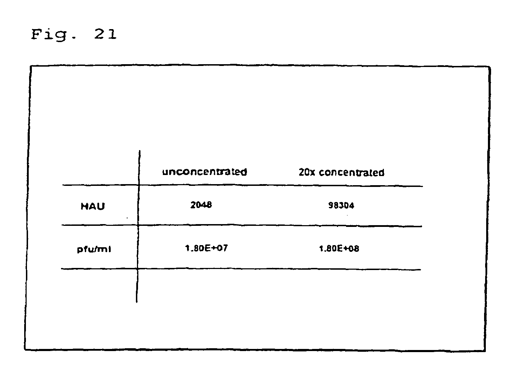

Suspension growing PER.C6 cells were incubated at 37°C in a 15 liter-bioreactor hollow fiber perfusion system, with a cell suspension volume of 10 liter in serum-free ExCell 525 medium (JRH Biosciences). Influenza infection was carried out at 35°C at a cellular density of approximately 3.3x10⁶ cells/ml in medium containing 5 µg/ml trypsin-EDTA (Life Technologies). Cells were inoculated with PER.C6-grown A/Sydney/5/97 virus (passage number 3) at an moi of 10⁻⁴ pfu/cell. Perfusion with serum-free ExCell 525 medium containing trypsin-EDTA was continued during the first 24 h upon infection. Two days post-infection, cells were fed with a fed-batch solution containing glucose, essential amino acids and extra glutamine: 82 ml per liter suspension containing 50m/v% glucose (NPBI-The Netherlands), 50x essential amino acids without Gln (Gibco-BRL-Life Technologies) and 200 mM glutamine (Gibco-BRL-Life Technologies). Cell suspension samples of 10 ml were taken every day in order to perform standard cell counts (results shown in Fig.17, left graph), glucose concentration measurements (results shown in Fig.17, right graph), direct immunofluorescence (Fig.18), haemagglutination (Fig.19) and infectivity assays (data not shown). Furthermore, the HA protein was investigated by western blot analysis and compared to a NIBSC standard HA control (Fig.20). On the day of the final harvest of the entire cell suspension (92 h post infection), a cell debris clarification was performed in a continuous flow at 20,000g using the Powerfuge™ separation system (Carr, JM Separations) according to the protocols provided by the manufacturer. Clarified supernatant was then concentrated twenty fold using a hollow fiber membrane cartridge of 500 kD cut off (A/G Technology, JM Separations). The results depicted in Fig.21 show that the quantitative recovery of live Influenza virus after concentration by hollow fiber as measured by haemagglutination and infectivity assays is very significant.

### Example 10

### The immunogenicity of PER.C6-grown Influenza viruses and vaccines derived thereof.

To determine the immunogenicity of PER.C6 grown Influenza viruses an *in vivo* study and challenging model in ferrets was designed. Two batches of trivalent whole-inactivated Influenza vaccine (composed of A/Sydney/5/97, A/Beijing/262/95 and B/Harbin/7/94), containing 15 µg HA of each of the three strains, were used. The first batch was obtained from fertile hens' eggs and the second was obtained from PER.C6 cells. Production, purification, inactivation and formulation of the trivalent whole-inactivated PER.C6-derived Influenza vaccines were performed as described below.

### Growth of A/Sydney/5/97, A/Beijing/262/95 and B/Harbin/7/94 Influenza strains on PER.C6.

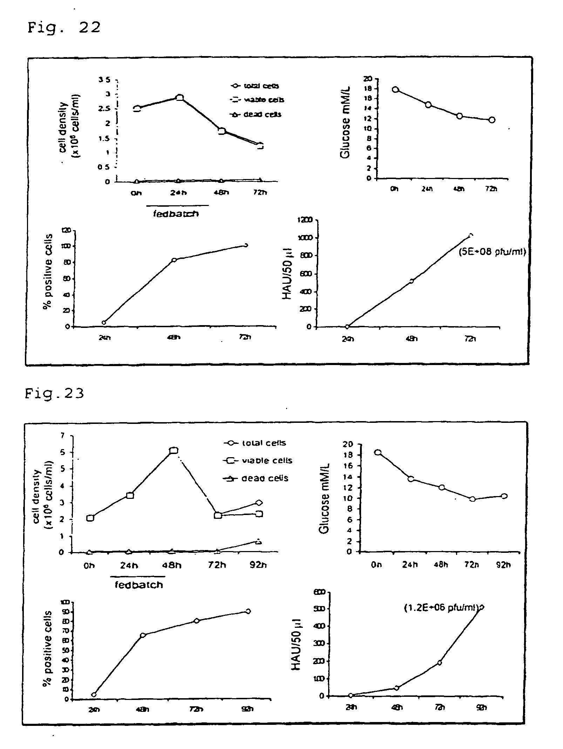

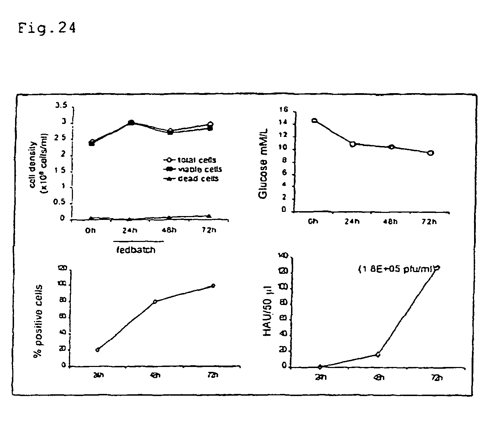

Production of all three Influenza viral batches were performed in three separate 3 liter hollow fiber fed-batch bioreactor systems with cell suspension volumes of 2 liter. Fedbatch was performed with the addition of the following solution: A total volume of 96 ml containing 50m/v% glucose (NPBI), 50x essential amino acids without Gln (Gibco-BRL-Life Technologies), 200 mM glutamine (Gibco-BRL-Life Technologies) and 7.5 m/v% NaHCO₃ (Merck) was added once. Influenza infections were carried out at cell densities ranging from 1.8x10⁶ to 2.6x10⁶ viable cells/ml, in ExCell 525 serum free medium containing 5 µg/ml trypsin-EDTA. PER.C6 cells were inoculated with the PER.C6-grown A/Sydney/5/97, A/Beijing/262/95 and B/Harbin/7/94 virus batches at different moi's: 10⁻⁴ (A/Sydney/5/97) or 10⁻³ (A/Beijing/262/95 and B/Harbin/7/94) pfu/cell. During the virus production period, samples of 10 ml were taken every day to perform standard cell and viability counts, glucose concentration measurements, direct immunofluorescence and Haemagglutination assays. Fig.22 (results from the A/Sydney/5/97-infected PER.C6 cells) shows the total and viability cell counts after infection with the virus (upper left panel), the glucose consumption (upper right panel), the percentage of positive cells in the direct immunofluorescence detection (lower left panel) and the HAU's (lower right panel). Fig.23 (results from the A/Beijing/262/95-infected PER.C6 cells) shows the total and viability cell counts after infection with the virus (upper left panel), the glucose consumption (upper right panel), the percentage of positive cells in the direct immunofluorescence detection (lower left panel) and the HAU's (lower right panel). Fig.24 (results from the B/Harbin/7/94-infected PER.C6 cells) shows the total and viability cell counts after infection with the virus (upper left panel), the glucose consumption (upper right panel), the percentage of positive cells in the direct immunofluorescence detection (lower left panel) and the HAU's (lower right panel). Virus containing concentrates were stored at -80°C until DSP.

In all three cases the glucose consumption and viability and total cell counts of the PER.C6 cells were comparable. Also the production levels of the three viruses, as measured by direct immunofluorescence were similar. Although the HAU and infectivity titers differed between different strains, PER.C6 sustained replication of all Influenza viruses that were tested here.

On the day of harvest of the entire batch (either at day 3 or at day 4 post-infection) viral supernatants were clarified by centrifugation at 2000 rpm in a table top centrifuge and concentrated ten fold by ultra filtration using a hollow fiber membrane cartridge of 750 kD cut-off (A/G Technology, JM Separations) following the protocols provided by the manufacturer. Influenza viruses were purified from the concentrated supernatants via two subsequent density centrifugation steps: a 25-70% block sucrose gradient (1.5hrs at 27K) followed by a continuous 25-70% sucrose gradient (4hrs at 23K). Viral bands were diluted in approximately 50 ml of a Phosphate buffer and finally pelletted at 24,000 rpm in an ultracentrifuge. Viral pellets were dissolved in 1.5 to 2.3 ml of a Phosphate buffer, aliquotted and frozen at -80°C. The formulation of inactivated Influenza vaccines is based on the amount (in micrograms) of the "immunologically active" HA protein, as measured by the SRID assay (Wood et al 1977). The test was performed to characterize the HA content of the batches. At the same time total amount of proteins was measured using the Lowry-based DC-protein assay kit (Biorad) following the procedures suggested by the manufacturer. It was found that HA constitutes about 20 to 30% of the total protein content of the virus preparation.

### Example 11

### In vivo Immunogenicity of Inactivated Vaccines Produced in Eggs and on PER.C6.

Ferrets and mice represent two well-established animal models to study Influenza infection and have been used to determine the efficacy and immunogenicity of Influenza vaccines. Using the mouse model test system, the immunogenicity produced by the PER.C6 and egg derived trivalent vaccines containing A/Sydney/5/97, A/Beijing/262/95 and B/Harbin/7/94 are compared by analyzing sera of vaccinated animals by Haemagglutination inhibition assay. Using the ferret infection model, immunization is followed by a challenge with A/Sydney/5/97. Virus recovery on MDCK cells and Haemagglutination inhibition assay performed on the sera are used to compare the immunogenicity and efficacy of the two vaccines.

### In vivo study in mice.

Ninety female Balb/C mice are divided into nine groups of ten mice. On day 0, up to 100 µl of blood is collected. The serum is separated and stored at -20°C. Each mouse is then vaccinated with the appropriate vaccine according to the schedule in Table I. On day 28, a further 100 µl of blood is taken. Serum is stored at -20°C. Each mouse is again vaccinated according to the schedule in Table I. On day 42, a 100 µl blood sample is taken and all mice are sacrificed. Serum is separated and frozen at -20°C. Haemagglutination Inhibition (HI) assays are conducted on serum samples from day 0, 28 and 42. All these assays are conducted in parallel for each day for both egg and cell grown viruses.

**Table I**

| Immunogenicity test in mice. | | | | |
|---|---|---|---|---|
| GROUP NUMBER | ANTIGEN TYPE | IMMUNIZATION VOLUME (ml) | VACCINATION ROUTE | TOTAL µg HA per dose |
| 1 | Egg trivalent whole virion | 0.5 | s.c. | 9.0 |
| 2 | Egg trivalent whole virion | 0.5 | s.c. | 3.0 |
| 3 | Egg trivalent whole virion | 0.5 | s.c. | 1.5 |
| 4 | Egg trivalent whole virion | 0.5 | s.c. | 0.15 |
| 5 | PER.C6 trivalent whole virion | 0.5 | s.c. | 9.0 |
| 6 | PER.C6 trivalent whole virion | 0.5 | s.c. | 3.0 |
| 7 | PER.C6 trivalent whole virion | 0.5 | s.c. | 1.5 |
| 8 | PER.C6 trivalent whole virion | 0.5 | s.c. | 0.15 |
| 9 | PBS | 0.5 | s.c. | 0 |

### In vivo study in ferrets.

Eighteen adult female ferrets (albino or polecat) were divided in three groups of six as follows: Group 1 received the egg derived test vaccine Intra Muscularly (IM), the animals were challenged with A/Sydney/5/97. Group 2 received the PER.C6 derived test vaccine IM, the animals were challenged with A/Sydney/5/97. Group 3 received the test vaccine diluent only and were challenged with A/Sydney/5/97. On days 0 and 28, the test vaccines were administered. On day 56, all the ferrets were infected intra-nasally with 0.5 ml of the A/Sydney/5/97 challenge virus at TCID₅₀ 10³. Nasal washes were performed and inflammatory cell counts, temperature and weights of the ferrets were monitored once daily from day 57 to 63. All animals were sacrificed on day 63. Serum was separated and stored at -20°C. The nasal wash samples were stored on ice and a nasal wash recovery cell count was performed using Trypan blue exclusion assay.

The titer of the virus obtained from the nasal wash samples was determined by measuring the virus recovery on MDCK cells. The Spearman and Karber (1931) calculation was used to calculate TCID₅₀ values. Haemagglutination Inhibition analysis were conducted on serum samples taken on day0, 28, 56 and 63. From this experiment it was concluded that the PER.C6 derived test vaccine was effective.

### Example 12

### Characterization of HA protein derived from Influenza virus produced on PER.C6.

In order to study the glycosylation of HA in PER.C6 cells, a batch of uncleaved HA (HAO) was generated. PER.C6 cells were infected with virus A/Sydney/5/97 (passage number 5 on PER.C6) at moi's of 1, 0.1 and 0.01 pfu/cell in ExCell 525 medium containing trypsin-EDTA at the final concentration of 5 µg/ml. After 1 h of incubation at 35°C, cells were washed twice with PBS to remove trypsin and incubated O/N at 35°C and 10% CO₂, in the absence of trypsin. The day thereafter, cell suspensions were harvested and centrifuged (500g) and cell pellets were washed twice with medium. Viral supernatants were frozen at -80°C and samples thereof were used in western blot assays as described to investigate the presence or absence of uncleaved HA protein.

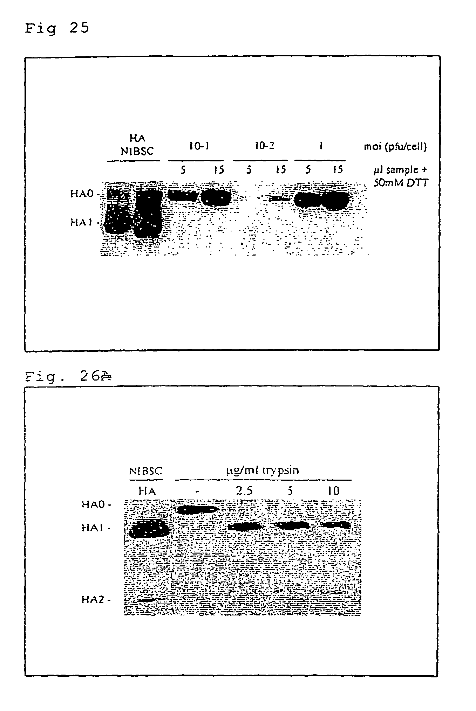

Uncleaved HA protein (HAO) consists of the two subunits: HA1 and HA2, that are connected via a disulfide bond. Since this disulfide bond can be disrupted by reduction with DTT, HA1 and HA2 can be separated and visualized on a reducing gel followed by western blots using antisera that recognize the subunits. If the HA protein consists only of HAO, one band will be visible that migrates slower through an SDS/PAGE gel as compared to the HA1 subunit and the fastest migrating HA2 subunit. The results shown in Fig.25 suggest the presence of mainly uncleaved HA0 from PER.C6 infections when compared to the egg-derived positive control that was obtained from the NIBSC (UK). To confirm that the band detected was indeed uncleaved haemagglutinin, digested an HA0 sample was digested with different concentrations of trypsin ranging from 2.5 to 10 µg/ml in medium O/N at 37°C. The digested proteins were then loaded under reducing conditions on an SDS/PAGE gel followed by western blot analysis using the same antisera as described for Fig.14. As shown in Fig.26A, cleavage of the HA0 could be achieved, confirming the generation of uncleaved HA protein on PER.C6.

### Example 13

### Digestion of HAO with N-Glycosidase F

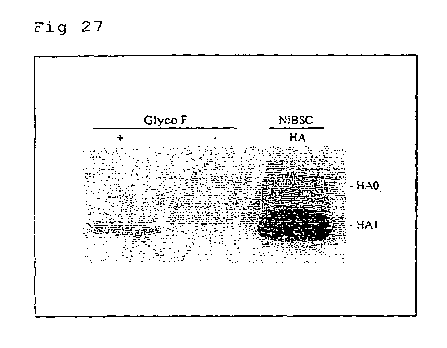

The Influenza virus HA protein is a glycoprotein that contains 3 to 9 N-linked glycosylation oligosaccharide sites. The number of sites depends on the virus strain. The location of these sites is determined by the nucleotide sequence of the HA gene, and since the viral genome of Influenza is replicated by an error-prone RNA polymerase, mutations that generate the addition or removal of glycosylation sites occur at high frequency. The composition and structure of the oligosaccharide chains present on the HA is then determined by the array of biosynthetic and trimming glycosylation enzymes provided by the host cell. Since glycosylation of HA plays an important role in virulence and vaccine efficacy, the properties of HA produced on Influenza infected PER.C6 was investigated. A digestion of A/Sydney/5/97 uncleaved HA0 protein with the N-glycosydase F enzyme was performed using protocols provided by the manufacturer to remove the seven oligosaccharides expected to be present on the A/Sydney/5/97 HA polypeptide. Influenza A/Sydney/5/97 was lysed with 1% Triton X-100 (Merck). Protease inhibitor was added to an aliquot of this lysed virus corresponding to 68 ng of HA, to a final concentration of lx (Complete protease inhibitor cocktail Boehringer Mannheim). This sample was incubated in the presence of 100 mM NaPO₄ pH 7, 10mM EDTA (J.T. Baker), 1% SDS (J.T. Baker) and 1% B-mercaptoehanol (Merck). This was incubated for 10 min at room temperature. The sample was diluted 5 times in mM NaPO₄ pH 7, 10mM EDTA (J.T. Baker), 0.625% NP-40 and 1% B-mercaptoehanol (Merck). Of this, 40 µl was used for the glyco-F digestion. For this 2 µl 1U/µl of glyco-F (N-Glycosidase F, Boehringer) was added and incubated for a minimum period of 16 h at 37°C. Then 3x Laemli buffer with 150 mM DTT (Fluka) was added to a final concentration of lx. The samples were run on a 7.5% SDS/PAGE gel. The SDS-Page and western blot were performed as follows. In brief, the blot was blocked for 30 min at room temperature with block solution (5% non fat dry milkpowder, Biorad in TBST suplemented with 1% rabbitserum (Rockland), followed by 3 washes with TBST. Then, the blot was incubated with the anti A/Sydney/5/97 HA antiserum (98/768 NIBSC) diluted 1/500 in 1%BSA/TBST with 5% rabbitserum (Rockland) overnight at roomtemperature. Again the blot was washed 8 times with TBST. Finally the blot was incubated with the rabbit anti sheep antiserum-HRP labeled (Rockland) 1/6000 diluted in blocksolution for 1 h at room temperature. After 8 washes with TBST the protein-conjugate complex was visualized with ECL (Amersham Pharmacia Biotech), and films (Hyperfilm, Amersham Life Science) were exposed. As shown in Fig.27, treatment with the glycosidase-F enzyme clearly reduced the size of the protein with approximately 28-30kD, being approximately the predicted loss of about 4 kD per oligosaccharide. The protein band depicted with an asterisks (*) is the de-glycosylated HA0, that migrates similarly to the HA1 subunit product obtained after cleavage of HA0 into HA1 and HA2 subunits (right lanes).

### Example 14

### Cleavage of HAO with Accutase.

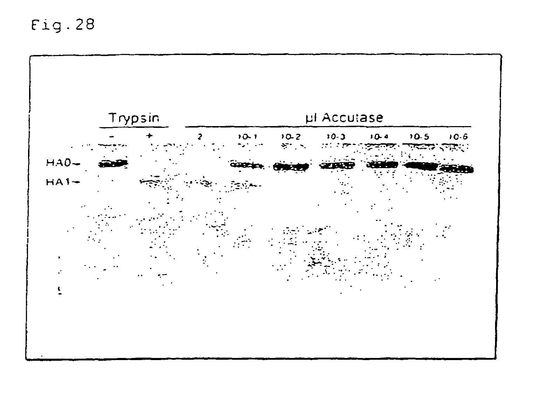

The possibility to replace the mammalian-derived trypsin-EDTA with non-mammalian or recombinant proteins was investigated. Recently, a mixture of proteolytic and collagenolytic enzymes (Accutase™, PAA) from invertebrate species became available for routine cell culture. Due to its non-mammalian source Accutase is free of prions, parvovirus and other components that potentially can contaminate trypsin-EDTA solutions. No information regarding the type of proteases present in Accutase could be obtained to date. The cleavage of HA0 was studied using western blot analysis. A constant amount of HA0 protein, obtained by PER.C6 infected with A/Sydney/5/97 at an moi 1 pfu per cell without trypsin, was digested with serial dilutions of Accutase, O/N at 37°C. As a positive control the same amount of HA0 digested with 100 ng of trypsin-EDTA was used. The digested proteins were then loaded on a 10% SDS-PAGE gel, under reducing conditions, for western blot analysis. As shown in Fig.28 digestion with 2 µl of Accutase treatment resulted in complete cleavage of HA0; partial cleavage was observed using 0.2µl.

These results suggest that treatment with Accutase during Influenza replication and production can replace trypsin-EDTA during Influenza infections on PER.C6.

### Example 15

### Electron microscopy analysis of Influenza viruses on PER.C6 cells.

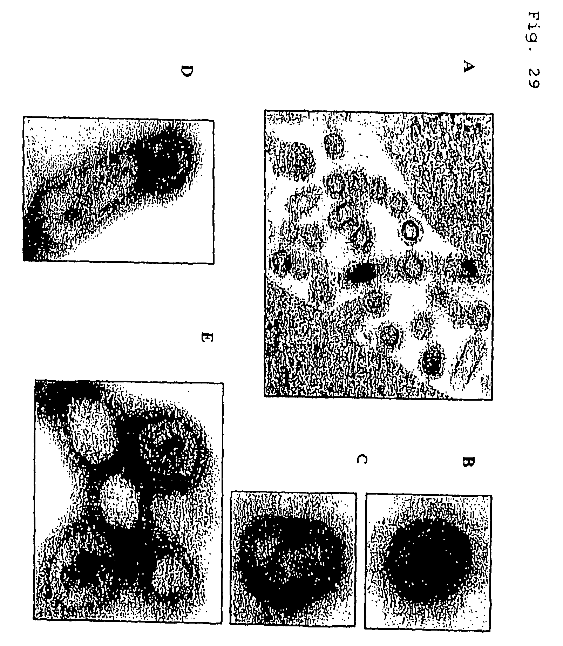

Transmission electron microscopy studies were done on PER.C6 cells that were infected with the Influenza strain A/Sydney/5/97 as well as on viral containing supernatants and sucrose purified material to determine the phenotype of this Influenza virus produced on PER.C6. All methods that were used are well known to persons skilled in the art. Fig.29 shows that the last stages of the virus life cycle are represented by budding and release of enveloped virions from the cytoplasmic membrane. Spikes corresponding to the HA and viral proteins were detected, ornamenting the periphery of the virion particles. The figure also shows the characteristic pleiomorphism of Influenza viruses.

### Example 16

### Infection of PER.C6 with a large variety of Infuenza A and B virus strains

The use of PER.C6 as a platform technology for the production of Influenza vaccine requires PER.C6 to support the growth of a wide range of strains of different Influenza subtypes.

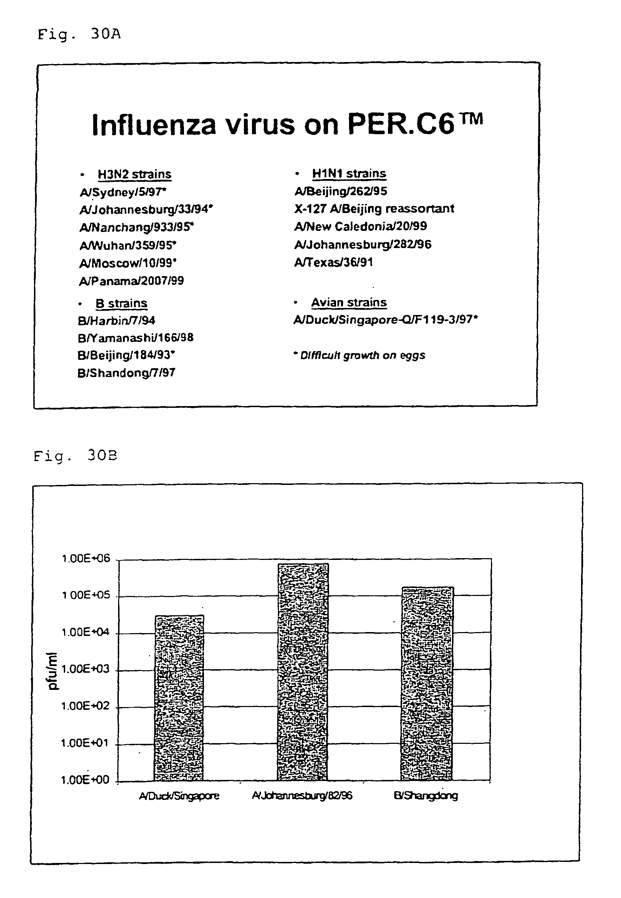

Static suspension cultures of PER.C6 cells that were grown in T25 flasks and/or in 6 well plates in ExCell 525 medium, were infected at a cell density of 10⁶ cells/ml with 16 different strains of Influenza viruses shown in Fig.30A. These strains comprised several H3N2, H1N1, B type and Avian strains. Infections were performed in the presence of 5 pg/ml of trypsin. The viruses were obtained from NIBSC (UK) as egg-passaged wild type or reassortant strains . Infection was performed with a virus dilution recommended by the NIBSC in the product sheets that were delivered with the different strains. All viruses tested were capable of propagation on PER.C6 as visualized by immunofluorescence (data not shown) and titration of supernatant fluids in pfu assay (Fig.30B).

These results show that even Influenza strains (depicted by an asterisks), such as A/Johannesburg/33/94, B/Beijing/184/93 and A/Duck/Singapore-Q/F119-3/97, that are normally very difficult to produce on embryonated eggs can replicate and be produced on the human PER.C6 cells.

### Example 17

### Generation of Herpes Simplex type 1 (HSV-1) virus, Herpes Simplex type 2 (HSV-2) virus and Measles virus on PER.C6.

It was tested whether other viruses than Influenza virus and Adenovirus, such as Herpes simplex virus type 1 and 2 and Measles virus could also replicate on PER.C6. Vaccines that are derived from these PER.C6-grown viruses and that induce neutralizing effects in humans for protection against wild type infections are generated from the PER.C6-grown virus batches. The strains that were obtained from ATCC and used for infection of PER.C6 cells are depicted in Table II.

**Table II**

| Herpes simplex virus and Measles strains that were obtained from the ATCC and that were used for infection of PER.C6 cells. | | | | | |
|---|---|---|---|---|---|
| **Virus** | **Strain** | **ATCC catnr.** | **Lotnr.** | **Passage history** | **Titer** |
| Herpes Simplex Type 1 | Macintyre | VR-539 | 1327850 | y.s./12, PR RabK/5, Mb/1, PrRabK/5, Vero/4, Vero(ATCC CCl-81)/1 | 10^{6.75} TCID50/200µl |
| Herpes Simplex Type 2 | MS | VR-540 | 216463 | Sheep choroid plexus/?, HeLa/?, PrRabK/7, Vero(ATCC CCl-81)/3 | 10^{7.5} TCID50/200µl |
| Measles | Edmonston | VR-24 | 215236 | HK/24, HuAm/40, MRC-5/1, MRC-5(ATCC CCL-171)/1 | 10⁴ TCID50/ml |

To test whether HSV-1 and HSV-2 and measles viruses obtained from the ATCC could replicate and be produced on PER.C6, passage number 46 cells were seeded in labtekchambers, coated with Poly-L-Lysine using methods known to persons skilled in the art, at 10⁵ cells/well. Monkey derived Vero cells (obtained from ATCC) were cultured at passage number 137 and were used as positive controls and seeded at a density of 2.5x10⁴ cells/well. At day 0, when wells with PER.C6 cells were 60% and Vero cells 80% confluent, cells were infected with different moi's ( 10⁻³, 10⁻², 10⁻¹ and 1 TCID₅₀ per cell) . At daily intervals upon infection, cells were fixed and assayed in immunofluorescence using FITC-conjugated type specific monoclonal antibodies using a kit (Imagen Herpes Simplex Virus (HSV) Type 1 and 2, (Dako) and FITC-conjugated antibodies against the HA and matrix protein of measles virus (measles IFA kit, Light diagnostics), following the procedures suggested by the manufacturer. The antisera are directed against HSV-1 and -2 and Measles virus antigens.

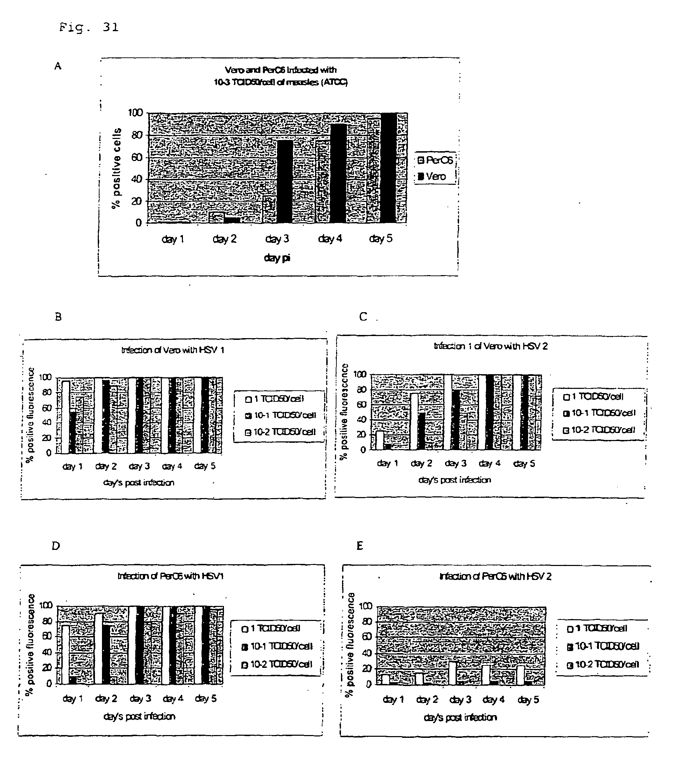

The results summarized in Fig.31 show that PER.C6 is permissive for HSV-1 (Fig.31D), HSV-2 (Fig.31E) and Measles virus (Fig.31A) infections. Furthermore, the kinetics suggest that these viruses replicate on PER.C6 in an moi-dependent manner.

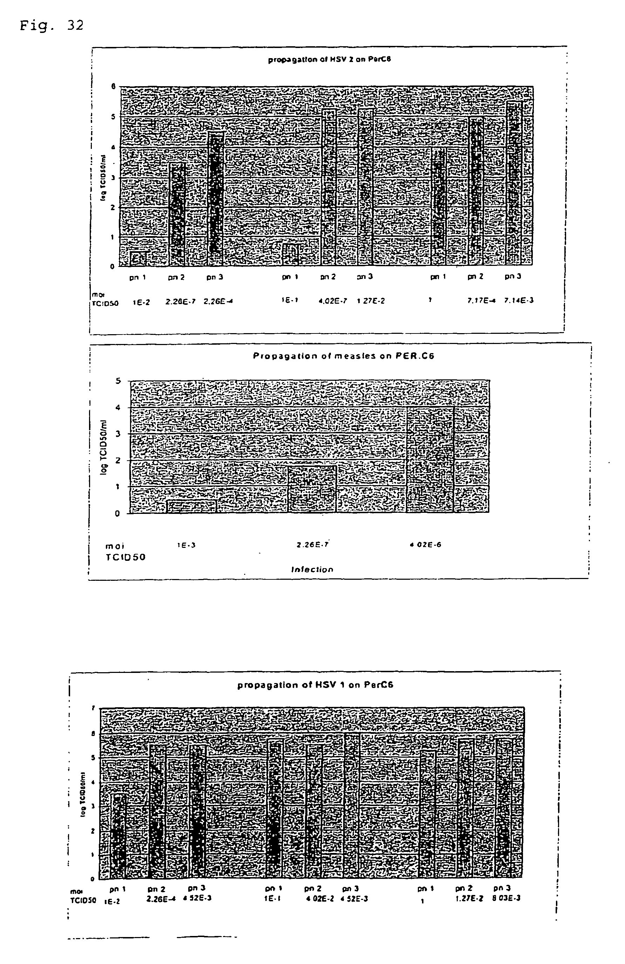

Next it was investigated whether HSV-1, -2 and Measles virus could be propagated on PER.C6. To this end cells were infected with moi of 0.01, 0.1 and 1 TCID₅₀/cell for HSV-1 (Fig.32C) and HSV-2 (Fig.32A) and an moi of 0.001 TCID₅₀/cell for Measles virus (Fig.32B) (passage number 1). At the occurrence of almost complete cpe, cells and supernatants were harvested, quickly frozen in liquid N₂, and thawed. After this, clarified supernatants were passaged blindly using approximately 100 µl, to PER.C6 (this is passage number 2). After reaching almost complete cpe again, a third passage (passage number 3) was performed in a similar manner. The moi's of the passage number 2 and 3 were determined in retrospect by TCID₅₀ assays.

The results of these experiments show that Herpes Simplex Virus type 1 and -2 and Measles viruses can be replicated on PER.C6 and that replication and propagation can even occur when moi's as low as 10⁻⁷ are used.

### Example 18

### Screening of rotavirus for replication on PER.C6.

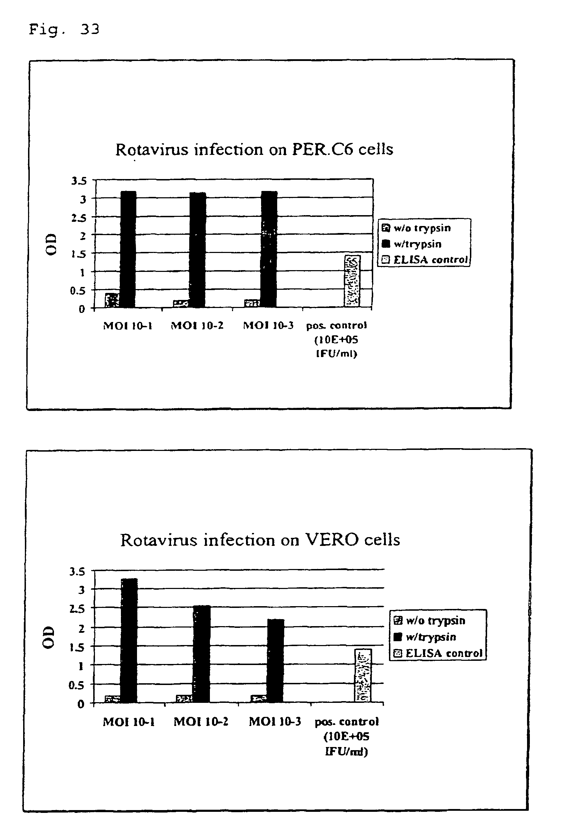

To test whether PER.C6 could also support the replication of a rotavirus, PER.C6 cells were infected with a Rhesus rotavirus (MMU 18006;ATCC#VR-954;strain S:USA:79:2; lot#2181153). PER.C6 cells (passage number 41) were cultured at a density of 1x10⁵ per ml and Monkey derived Vero cells (obtained from ATCC, passage number 139) were cultured at a density of 2.5x10⁴ per ml, and subsequently seeded in Labtekchambers, that had been pre-coated with poly-L-Lysine using methods known to persons skilled in the art. Cells were infected with an moi of 1 TCID₅₀/cell of Rhesus rotavirus in the presence and absence of 2 µg/ml of trypsin-EDTA. After 90 min of infections, cells were washed with ExCell 525 medium and further incubated at 37°C at 10% CO₂ in a humidified atmosphere. On 5 consecutive days following infection, samples of supernatants were harvested, clarified from cells and cell debris by centrifugation at 2000 rpm in a table top centrifuge and analysed in an ELISA specific for rotavirus (IDEIA Rotavirus, Dako). The results depicted in Fig.33 clearly show that Rhesus rotavirus replicates on PER.C6.

### LEGENDS TO THE FIGURES

Fig.1. Percentage of infected cells (positive cells) viewed microscopically after immunofluorescence assay versus percentage of dead cells measured via FACS after propidium iodide staining, at moi's of 10⁻³ and 10⁻⁴.
   Poor viability of the cells from samples derived from infection at moi 10⁻³ didn't give rise to reliable data.

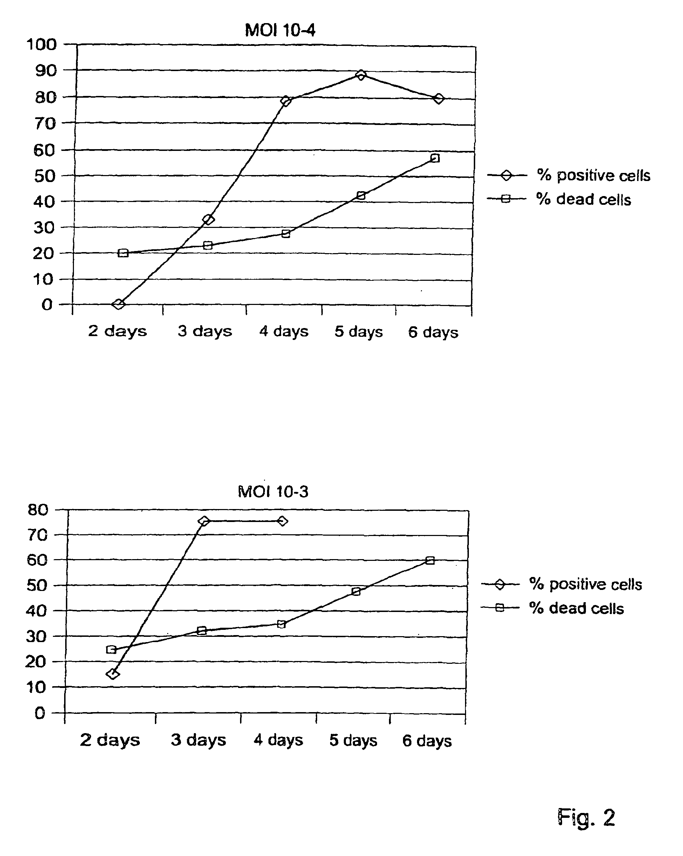

Fig.2. Percentage of infected cells viewed microscopically after immunofluorescence assay. Samples derived from infection at moi 10 and 1, at 48h post infection are not shown, because of full CPE

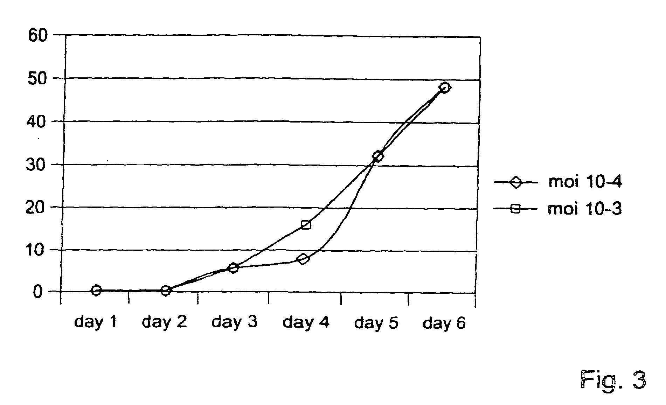

Fig.3. Kinetics of virus propagation measured in hemagglutinating units (HAU) from day 1 to day 6 after infection.
Fig.4. Percentage of infected cells (positive cells) viewed microscopically after immunofluorescence assay.
Fig.5. Kinetics of virus propagation measured in hemagglutinating units (HAU) from day 1 to 6 after infection.
Fig.6. Percentage of infected cells (positive cells) viewed microscopically after immunofluorescence assay.
Fig.7. Kinetics of virus propagation measured in hemagglutinating units (HAU) from day 2 to 6 after infection.
Fig.8. Expression of Sia2-3Gal and Sia2-6Gal linkages on cell surface receptors present on Chinese Hamster Ovary (CHO) cells, PER.C6 cells and MDCK cells. (A) Schematic representation of the interaction of the Sambuca nigra agglutinin (SNA) lectin that specifically recognizes Sia2-6Gal linkages and the Maackia amurensis agglutinin (MAA) lectin that specifically recognizes Sia2-3Gal linkages. The schematic interaction with the FITC labelled anti-DIG antibody recognizing the DIG labelled lectin bound to the oligosaccharide chain on the cell surface protein is also depicted. (B) FACS analysis of cells incubated with DIG-labeled lectins. Lectins attached to the cells were detected with FITC-labeled anti-DIG antibody using procedures known to persons skilled in the art. Cell number counts are plotted against the fluorescence intensity of lectin-stained cells (gray) as compared with cells that were incubated only with the FITC-anti-DIG antibody (open). The upper panels show the shift in the FACS analysis obtained by using the SNA lectin and the lower panels show the shift in the FACS analysis obtained by using the MAA lectin.
Fig.9. Infection with A/Sydney/5/97 on PER.C6. (A) Effect of trypsin-EDTA on HAU titers. (B) HA concentration in µg/ml and (C) virus infectivity titers in pfu's/ml as measured in crude viral supernatants, 96 hours post infection.
Fig.10. Infection with B/Harbin/7/94 on PER.C6. (A) Effect of different concentrations of trypsin-EDTA present during and after virus infection on growth kinetics. (B) HAU titers per 50 µl and (C) virus infectivity titers in pfu/ml.
Fig.11. Infection with X-127 using an moi of 10⁻³ on PER.C6. (A) Effect of trypsin-EDTA on HAU given in HAU/50 µl and (B) virus infectivity titers in pfu/ml during 5 days after infection.
Fig.12. Infection with X-127 using an moi of 10⁻⁴ on PER.C6. (A) Effect of trypsin-EDTA on HAU given in HAU/50 µl and (B) virus infectivity titers in pfu/ml during 5 days after infection.
Fig.13. Effect of trypsin-EDTA on (A) PER.C6 cells viability and (B) biological activity of the virus. Cell viability was measured after trypan-blue staining. HAU titers were measured as described and given per 50 µl.
Fig.14. Effect of trypsin-EDTA on virus infectivity titers and HA protein content after Influenza infection of PER.C6 cells with A/Sydney/5/97. (A) The infectivity assay was carried out by inoculating, in quadruplicate, MDCK cells with a total of 100 µl of 10-fold serially diluted virus containing supernatants, in serum free medium with trypsin-EDTA (4 µg/ml). After seven days, supernatant of these cultures were tested for HA activity. The infectious virus titers were calculated according to the method of Spearman-Karber (1931). (B) Western blot analysis of the A/Sydney/5/97 HA protein. Harvesting of viral proteins were carried out by disruption and denaturation of proteins using an SDS containing lysis buffer. The electrophoretic run was performed on a 10% SDS/PAGE gel under reducing conditions. Separated proteins were probed with the specific anti-A/Sydney-HA antisera. Increasing amounts of the positive control A/Sydney HA antigen (left 4 lanes) and 10 µl of PER.C6 cells supernatants of the indicated trypsin incubated samples (right 5 lanes) were loaded.
Fig.15. PER.C6 cells viability, glucose concentration and growth kinetics of A/Sydney/5/97 in a hollow fiber perfusion system.
Fig.16. Characterization and quantification of Influenza virus A/Sydney/5/97 propagated on PER.C6 in a hollow fiber perfusion system. SDS-PAGE and Western blots were done as described in legend to Fig.14 for the Sheep anti-A/Sydney-HA antibody. The monoclonal antibody anti HA-tag (HA probe (F7), mouse monoclonal, (Santa Cruz) was used in 1:1000 dilution. As a second antibody a goat anti mouse-HRP conjugated antibody (Biorad), in 1:7500 dilution was used.
Fig.17 PER.C6 cells viability (left panel) and glucose concentration (right panel) in a 12 liter bioreactor up to 92 h after viral infection using A/Sydney/5/97 virus.
Fig.18. Infection of PER.C6 with A/Sydney/5/97 in a 10 liter cells suspension in a 12 liter Bioreactor. Kinetics of virus replication as measured by Immunofluorescence assay are given in percentages of positively stained cells.
Fig.19. Infection of PER.C6 cells with A/Sydney/5/97 in a 10 liter cell suspension in a 12 liter Bioreactor. Kinetic of virus replication as measured by Haemagglutination assay are given in HAU's during several days after viral infection. The bar depicted with an asterisk is the number of HAU's obtained after Powerfuge^{TM} clarification as described in the text.
Fig.20. Western blot following an infection of PER.C6 with A/Sydney/5/97 virus in a 10 liter cell suspension in a 12 liter Bioreactor. Shown is the characterization and quantification of the Influenza virus A/Sydney/5/97 HA polypeptide. SDS/PAGE and Western blot were done as described in legend to Fig.14. The different subunits (HA1 and HA2) and the non-cleaved HA0 proteins are depicted by arrow heads. The HA obtained from NIBSC served as a positive control.
Fig.21. Determination of HAU's and pfu/ml after infection of PER.C6 with A/Sydney/5/97 in a 10 liter cell suspension in a 12 liter bioreactor. The infection was followed by Down Stream Processing (DSP). The recovery of viral yields after hollow fiber ultrafiltration (20 fold concentration) is also shown.
Fig.22. Infection of PER.C6 with A/Sydney/5/97 in a 2 liter cell suspension in a 3 liter bioreactor. PER.C6 cells viability (upper left), glucose concentration (upper right) and growth kinetics of the virus in the percentage of positively staining cells (lower left) and HAU's (lower right) are given.
Fig.23. Infection of PER.C6 with A/Beijing/262/95 in a 2 liter cell suspension in a 3 liter bioreactor. PER.C6 cells viability (upper left), glucose concentration (upper right) and growth kinetics of the virus in the percentage of positively staining cells (lower left) and HAU's (lower right) are given.
Fig.24. Infection of PER.C6 with B/Harbin/7/94 in a 2 liter cell suspension in a 3 liter Bioreactor. PER.C6 cells viability (upper left), glucose concentration (upper right) and growth kinetics of the virus in the percentage of positively staining cells (lower left) and HAU's (lower right) are given.
Fig.25. Western blot analysis of uncleaved A/Sydney/5/97 HA0 protein. Positive staining proteins are detected after incubation with the specific anti-A/Sydney antisera obtained from NIBSC and described as in the legend of Fig.14 and in the text.
Fig.26. Western blot analysis of A/Sydney/5/97 derived HA0 protein digested with trypsin. Proteins are detected after incubation with the specific anti-A/Sydney antisera. On the left a standard cleaved A/Sydney HA, on the right HA0 treated with increasing amount of trypsin.
Fig.27. Western blot analysis of A/Sydney HA0 digested with N-glycosydase F. Proteins are detected after incubation with the specific anti-A/Sydney antisera. The protein band depicted with an asterisk is the de-glycosylated product.
Fig.28. Western blot analysis of A/Sydney/5/97 HA after Accutase digestion. Proteins are detected after incubation with the specific policlonal anti-A/Sydney-HA antisera. On the left, HA0 before and after trypsin treatment, on the right HA0 digested with decreasing amount of Accutase.
Fig.29. Electron micrographs of Influenza A/Sydney/5/97. (A) PER.C6 cells 72 hrs post infection. (B and C) Negative staining on virus derived from infected PER.C6. (D and E) Negative staining of sucrose purified material.
Fig.30. (A) All different Influenza A and B strains tested on PER.C6 cells. (B) Infectivity titers of three depicted A- and B-type Influenza viruses derived from infected PER.C6 cells.
Fig.31. Immunofluorescence of PER.C6 and Vero cells infected with viruses other than Influenza. (A) Positively staining cells upon infection with Measles virus. (B) Positively staining cells upon infection of Vero cells with HSV-1 virus. (C) Positively staining cells upon infection of Vero cells with HSV-2 virus. (D) Positively staining cells upon infection of PER.C6 cells with HSV-1 virus. (E) Positively staining cells upon infection of PER.C6 cells with HSV-2 virus.
Fig.32. Infectivity titers determined after propagation of Measles virus (middle panel), HSV-1 (bottom panel) and HSV-2 (top panel) virus on PER.C6 cells.
Fig.33. Replication of Rotavirus after infection of PER.C6 (top panel) and Vero (bottom panel) cells with different moi's as measured by ELISA in crude supernatants.

### REFERENCES

Bachmayer H. Selective solubilisation of haemagglutinin and neuraminidase from Influenza virus. Intervirology 1975; 5:260-272.
Brands R, Palache AM, van Scharrenburg GJM. Madin Darby Canine Kidney (MDCK) -cells for the production of inactivated Influenza subunit vaccine. Safety characteristics and clinical results in the elderly. In: Brown LE, Hampson EW, Webster RG, editors. Option for the control of Influenza III. Amsterdam Elsevier, 1996. P. 683-693.
Brands R, Palache AM, van Scharrenburg GJM. Development of Influenza subunit vaccine produced using mammalian cell culture technology. In. Carrondo MJT, Griffths B, Moreira JLP, editors. Animal cell technology: from vaccines to genetic medicine. Dordrecht: Kluwer Academic Publishers, 1997:165-167.
Gubareva LV, Wood JM, Meyer WJ, Katz JM, Robertson JS, Major D, Webster RG. Codominant mixtures of viruses in strains of Influenza virus due to host cell variation. Virol. 1994; 199: 89-97.
Govorkova EA, Matrosovich MN, Tuzikov AB, Bovin NV, Gerdil C, Fanget B, Webster RG. Selection of receptor-binding variants of human Influenza A and B viruses in baby hamster kidney cells. Virology 1999 15;262(1):31-8
Herrero-Euribe L et al. Replication of Influenza A and B viruses in human diploid cells. J. Gen. Virol. 1983; 64: 471-475.
Karber G. Beitrag zur kollektiven behandlung pharmakologischer reihenversuche. Exp. Pathol. Pharmakol. 1931;162,480-483.
Kodihalli S, Justewicz DM, Gubareva LV, Webster RG. Selection of a single amino acid substitution in the haemagglutinin molecule by chicken eggs can render Influenza A virus (H3) candidate vaccine ineffective. J. Virol. 1995;69:4888-4897.
Kirstner O, Muller K, Scholtissek C. Differential phosphorylatian of the nucleoprotein of Influenza A viruses. J. Gen. Virol. 19989;70:2421-2431.
Marsha A. et al. Pharmacotherapy 19 (11):1279-1295, 1999: Rotavirus disease and its prevention in infants and children.
Murphy BR and Webster RG. Orthomyxoviruses. In: Fields Virology, chapter 46, 1397. Eds.B.N. Fields, D.M. Knipe, P.M. Howley, et al. Lippincott-Raven Publishers, Philadelphia 1996.
Newman RW, Jenning R Major DL, Robertson JS, Jenkins R, Potter CW, Burnett I, Jewes L, Anders M, Jackson D, Oxford JS. Immune response of human volunteers an animals to vaccination with egg grown Influenza A (H1N1) virus is influenced by three amino acid substitutions in the haemagglutinin molecule. Vaccine 1993;11:400-406.
Palache AM, Brands R, van Scharrenburg GJM. Immunogenecity and reactogenecity of Influenza subunit vaccines produced in MDCK cells or fertilized chicken eggs. J. Infect. Dis. 1977;176:S20-S23.
Robertson JS, Cook P, Nicolson C, Newman R,Wood JM. Mixed populations in Influenza vaccine strains. Vaccine 1994; 12:1317-1320.
Robertson JS,Bootman JS,Nicolson C, Mjor D, Robertson EW, Wood JM. The haemagglutinin of Influenza B virus present in clinical material is a single species identical to that of mammalian cell grown-virus. Virol. 1990; 179:35-40
Robertson JS, Bootman JS, newman R, Oxford JS, Daniels RS, Webster RG, Schild GC. Structural changes in the haemagglutinin which accompany egg adaptation of an Influenza A(H1N1) virus. Virol. 1987;160:31-37.
Schild GC, Oxford JS, de Jong JC, Webster RG. Evidence for host-cell selection of Influenza virus antigenic variants. Nature 1983; 303:706-709.
Schulman JL, Palese P. Virulence factors of Influenza A viruses: WSN virus neuraminidase required for plaque production in MDBK cells. J. Virol. 1977;24:170-176.
Sugiara A, Ueda M. Neurovirulence of Influenza virus in mice. I. Neurovirulence of recombinants between virulent and avirulent virus strains. Virol 1980;101:440-449.,495,271).
Tobita K, Sugiura A, Enomoto C, Furuyama M. Plaque assay and primary isolation of Influenza A viruses in an established line of canine Kidney cells (MDCK) in the presence of trypsin. Med.Microbiol. Immunol. 1975;162:9-14.
Williams SP, Robertson JS. Analysis of restriction to the growth of non-egg-adapted human Influenza in eggs. Virol. 1993;196:660665.
Wood JM, Schild GC, Newmann RW, Seagroatt V. Application of an improved single-radial-immunodiffusion technique for the assay of haemagglutinin antigen content of whole virus and subunit Influenza vaccines. Dev. Biol. Stand. 1977 1-3;39:193-20

## Claims

1. A method for producing a virus and/or viral proteins other than adenovirus or adenoviral proteins for use as a vaccine comprising providing a cell having been provided with at least a sequence encoding at least one gene product of the E1 gene or a functional derivative thereof of an adenovirus, with a nucleic acid encoding said virus, culturing said cell in a suitable medium and allowing for expression of said virus and harvesting said virus and/or viral proteins from said medium and/or said cell, wherein said cell is immortalised in order to grow in culture, and wherein said cell is derived from a human embryonic retinoblast.

2. A method according to claim 1, wherein said sequence encoding at least one gene product of the E1 gene is present in the genome of said human cell.

3. A method according to claim 1 or 2, wherein said cell does not produce adenoviral structural proteins.

4. A method according to any one of the aforegoing claims, wherein said cell further comprises a sequence encoding E2A or a functional derivative or analogue or fragment thereof.

5. A method according to any one of the aforegoing claims wherein said sequence encoding E2A or a functional derivative or analogue or fragment thereof is present in the genome of said human cell.

6. A method according to any one of claims 4 or 5, wherein said E2A encoding sequence encodes a temperature sensitive mutant E2A.

7. A method according to any one of the aforegoing claims, whereby said human cell comprises no other adenoviral sequences.

8. A method according to any one of the aforegoing claims, wherein said human cell is capable of growing in suspension.

9. A method according to anyone of the aforegoing claims wherein said human cell is cultured in the absence of serum.

10. A method according to any one of the aforegoing claims wherein said human cell is a cell as deposited under ECACC no. 96022940 or a derivative thereof.

11. A method according to any one of claims 1-10, wherein said virus comprises a protein that undergoes post-translational and/or peritranslational modifications.

12. A method according to claim 11 wherein said modifications comprise glycosylation.

13. A method according to claim 11 or 12 wherein said viral protein comprises at least one of an Influenza virus neuraminidase and/or a haemagglutinin.

14. A method according to any one of claims 1-12, wherein said virus is an enterovirus, such as rhinovirus, aphtovirus, or poliomyelitisvirus.

15. A method according to any one of claims 1-12, wherein said virus is a herpesvirus, such as herpes symplex virus, pseudorabies virus or bovine herpes virus.

16. A method according to any one of claims 1-12, wherein said virus is an orthomyxovirus, such as Influenza virus, a paramyxovirus, such as newcastle disease virus, respiratory syncitio virus, mumps virus or a measles virus.

17. A method according to any one of claims 1-12, wherein said virus is a retrovirus, such as human immunodeficiency virus or wherein said virus is a parvovirus or a papovavirus.

18. A method according to any one of claims 1-12, wherein said virus is a rotavirus or a coronavirus, such as transmissable gastroenteritisvirus or a flavivirus, such as tick-borne encephalitis virus or yellow fever virus.

19. A method according to any one of claims 1-12, wherein said virus is a togavirus, such as rubella virus or eastern-, western-, or venezuelean equine encephalomyelitis virus.

20. A method according to any one of claims 1-12, wherein said virus is a hepatitis causing virus, such as hepatitis A or hepatitis B virus.

21. A method according to any one of claims 1-12, wherein said virus is a pestivirus, such as hog cholera virus or a rhabdovirus, such as rabies virus.

22. A method according to any one of claims 1-12, wherein said virus is a Bunyaviridae virus, such as Hantavirus.

23. Use of an immortalised human cell having a sequence encoding at least one E1 protein of an adenovirus or a functional derivative, homologue or fragment thereof in its genome which cell does not produce structural adenoviral proteins for the production of a non-adenoviral virus for use in a vaccine, wherein said human cell is derived from a human embryonic retinoblast.

24. Use according to claim 23, wherein said human cell is a cell as deposited under ECACC no. 96022940 or a derivative thereof.

25. Use according to claim 23-24, wherein said human cell further comprises a sequence encoding E2A or a functional derivative or analogue or fragment thereof in its genome.

26. Use according to claim 25, wherein said E2A is temperature sensitive.

27. A human cell having a sequence encoding at least one E1 protein of an adenovirus or a functional derivative, homologue or fragment thereof in its genome, which cell does not produce structural adenoviral proteins and having a nucleic acid encoding a non-adenoviral virus, wherein said human cell is derived from a human embryonic retinoblast.

28. A human cell according to claim 27 which is derived from a cell as deposited under ECACC no. 96022940.

29. A human cell according to claim 27-28, which further comprises a sequence encoding E2A or a functional derivative or analogue or fragment thereof in its genome.

30. A human cell according to claim 29, wherein said E2A is temperature sensitive.

## Patentansprüche

1. Verfahren zur Herstellung eines Virus und/oder von viralen Proteinen, die von Adenovirus bzw. adenoviralen Proteinen verschieden sind, zur Verwendung als Impfstoff, umfassend das Versehen einer Zelle, die mit wenigstens einer Sequenz versehen wurde, die wenigstens ein Genprodukt des E1-Gens oder ein funktionelles Derivat davon eines Adenovirus codiert, mit einer Nucleinsäure, die das Virus codiert, das Züchten der Zelle in einem geeigneten Medium und die Ermöglichung der Expression des Virus und das Ernten des Virus und/oder der viralen Proteine aus dem Medium und/oder aus der Zelle, wobei die Zelle immortalisiert ist, um in Kultur zu wachsen, und wobei die Zelle von einem menschlichen embryonalen Retinoblasten abgeleitet ist.

2. Verfahren gemäß Anspruch 1, wobei die Sequenz, die wenigstens ein Genprodukt des E1-Gens codiert, in dem Genom der menschlichen Zelle vorhanden ist.

3. Verfahren gemäß Anspruch 1 oder 2, wobei die Zelle keine adenoviralen Strukturproteine produziert.

4. Verfahren gemäß einem der vorstehenden Ansprüche, wobei die Zelle weiterhin eine Sequenz umfasst, die E2A oder ein funktionelles Derivat oder Analogon oder Fragment davon codiert.

5. Verfahren gemäß einem der vorstehenden Ansprüche, wobei die Sequenz, die E2A oder ein funktionelles Derivat oder Analogon oder Fragment davon codiert, im Genom der menschlichen Zelle vorhanden ist.

6. Verfahren gemäß einem der Ansprüche 4 oder 5, wobei die E2A codierende Sequenz eine temperaturempfindliche E2A-Mutante codiert.

7. Verfahren gemäß einem der vorstehenden Ansprüche, wobei die menschliche Zelle keine weiteren adenoviralen Sequenzen umfasst.

8. Verfahren gemäß einem der vorstehenden Ansprüche, wobei die menschliche Zelle in Suspension wachsen kann.

9. Verfahren gemäß einem der vorstehenden Ansprüche, wobei die menschliche Zelle in Abwesenheit von Serum gezüchtet wird.

10. Verfahren gemäß einem der vorstehenden Ansprüche, wobei die menschliche Zelle eine Zelle wie hinterlegt unter ECACC-Nr. 96022940 oder ein Derivat davon ist.

11. Verfahren gemäß einem der Ansprüche 1 bis 10, wobei das Virus ein Protein umfasst, das posttranslationalen und/oder peritranslationalen Modifikationen unterliegt.

12. Verfahren gemäß Anspruch 11, wobei die Modifikationen Glykosylierung umfassen.

13. Verfahren gemäß Anspruch 11 oder 12, wobei das virale Protein wenigstens eines von Influenzavirus-Neuraminidase und/oder -Hämagglutinin umfasst.

14. Verfahren gemäß einem der Ansprüche 1 bis 12, wobei das Virus ein Enterovirus, wie ein Rhinovirus, Aphtovirus oder Poliomyelitisvirus ist.

15. Verfahren gemäß einem der Ansprüche 1 bis 12, wobei das Virus ein Herpesvirus, wie Herpes-simplex-Virus, Pseudorabiesvirus oder Rinderherpesvirus ist.

16. Verfahren gemäß einem der Ansprüche 1 bis 12, wobei das Virus ein Orthomyxovirus, wie ein Influenzavirus, ein Paramyxovirus, wie ein Newcastle-Virus, ein Respiratory-Syncytial-Virus, ein Mumpsvirus oder ein Masernvirus ist.

17. Verfahren gemäß einem der Ansprüche 1 bis 12, wobei das Virus ein Retrovirus, wie ein menschliches Immunschwächevirus (HIV), ist, oder wobei das Virus ein Parvovirus oder ein Papovavirus ist.

18. Verfahren gemäß einem der Ansprüche 1 bis 12, wobei das Virus ein Rotavirus oder ein Coronavirus, wie ein übertragbares Gastroenteritisvirus, oder ein Flavivirus, wie ein Zeckenencephalitisvirus oder Gelbfiebervirus, ist.

19. Verfahren gemäß einem der Ansprüche 1 bis 12, wobei das Virus ein Togavirus, wie Rubellavirus oder Östliches, Westliches oder Venezuelanisches Pferde-Encephalomyelitisvirus, ist.

20. Verfahren gemäß einem der Ansprüche 1 bis 12, wobei das Virus ein Hepatitis verursachendes Virus, wie Hepatitis-A- oder Hepatitis-B-Virus, ist.

21. Verfahren gemäß einem der Ansprüche 1 bis 12, wobei das Virus ein Pestivirus, wie ein Schweinepestvirus, oder ein Rhabdovirus, wie ein Rabiesvirus, ist.

22. Verfahren gemäß einem der Ansprüche 1 bis 12, wobei das Virus ein Bunyaviridae-Virus, wie ein Hantavirus, ist.

23. Verwendung einer immortalisierten menschlichen Zelle, die eine Sequenz, die wenigstens ein E1-Protein eines Adenovirus oder ein funktionelles Derivat, Homolog oder Fragment davon codiert, in ihrem Genom aufweist, wobei die Zelle keine adenoviralen Strukturproteine produziert, für die Produktion eines Nichtadenovirus zur Verwendung in einem Impfstoff, wobei die menschliche Zelle von einem menschlichen embryonalen Retinoblasten abgeleitet ist.

24. Verwendung gemäß Anspruch 23, wobei die menschliche Zelle eine Zelle wie hinterlegt unter ECACC-Nr. 96022940 oder ein Derivat davon ist.

25. Verwendung gemäß Anspruch 23 bis 24, wobei die Zelle weiterhin eine Sequenz, die E2A oder ein funktionelles Derivat oder Analogon oder Fragment davon codiert, in ihrem Genom umfasst.

26. Verwendung gemäß Anspruch 25, wobei das E2A temperaturempfindlich ist.

27. Menschliche Zelle, die eine Sequenz, die wenigstens ein E1-Protein eines Adenovirus oder ein funktionelles Derivat, Homolog oder Fragment davon codiert, in ihrem Genom aufweist, wobei die Zelle keine adenoviralen Strukturproteine produziert, und eine Nucleinsäure, die ein Nichtadenovirus codiert, wobei die menschliche Zelle von einem menschlichen embryonalen Retinoblasten abgeleitet ist.

28. Menschliche Zelle gemäß Anspruch 27, die von einer Zelle wie hinterlegt unter ECACC-Nr. 96022940 abgeleitet ist.

29. Menschliche Zelle gemäß Anspruch 27 bis 28, die weiterhin eine Sequenz, die E2A oder ein funktionelles Derivat oder Analogon oder Fragment davon codiert, in ihrem Genom umfasst.

30. Menschliche Zelle gemäß Anspruch 29, wobei das E2A temperaturempfindlich ist.

## Revendications

1. Procédé pour produire un virus et/ou des protéines virales autres qu'un adénovirus ou protéines adénovirales pour une utilisation en tant que vaccin, consistant à fournir une cellule ayant été munie d'au moins une séquence codant au moins un produit génique du gène E1 ou d'un dérivé fonctionnel de celui-ci d'un adénovirus, avec un acide nucléique codant ledit virus, à mettre en culture ladite cellule dans un milieu approprié et à laisser s'exprimer ledit virus et à recueillir ledit virus et/ou lesdites protéines virales à partir dudit milieu et/ou de ladite cellule, dans lequel ladite cellule est immortalisée de façon à croître en culture, et dans lequel ladite cellule est dérivée d'une cellule embryonnaire de la rétine humaine.

2. Procédé selon la revendication 1, dans lequel ladite séquence codant au moins un produit génique du gène E1 est présente dans le génome de ladite cellule humaine.

3. Procédé selon la revendication 1 ou 2, dans lequel ladite cellule ne produit pas de protéines structurelles adénovirales.

4. Procédé selon une quelconque des revendications ci-dessus, dans lequel ladite cellule comprend de plus une séquence codant E2A ou un dérivé ou analogue ou fragment fonctionnel de celui-ci.

5. Procédé selon une quelconque des revendications ci-dessus, dans lequel ladite séquence codant E2A ou un dérivé ou analogue ou fragment fonctionnel de celui-ci est présente dans le génome de ladite cellule humaine.

6. Procédé selon une quelconque des revendications 4 ou 5, dans lequel ladite séquence codant E2A code un mutant E2A sensible à la température.

7. Procédé selon une quelconque des revendications ci-dessus, dans lequel ladite cellule humaine comprend aucune autre séquence adénovirale.

8. Procédé selon une quelconque des revendications ci-dessus, dans lequel ladite cellule humaine est capable de croître en suspension.

9. Procédé selon une quelconque des revendications ci-dessus, dans lequel ladite cellule humaine est mise en culture en l'absence de sérum.

10. Procédé selon une quelconque des revendications ci-dessus, dans lequel ladite cellule humaine est une cellule telle que déposée à l'ECACC sous le numéro 96 022 940 ou un dérivé de celle-ci.

11. Procédé selon une quelconque des revendications 1-10, dans lequel ledit virus comprend une protéine qui subit des modifications post-traductionnelles et/ou péritraductionnelles.

12. Procédé selon la revendication 11, dans lequel lesdites modifications comprennent la glycosylation.

13. Procédé selon la revendication 11 ou 12, dans lequel ladite protéine virale comprend au moins une neuraminidase du virus de la grippe et/ou une hémagglutinine.

14. Procédé selon une quelconque des revendications 1-12, dans lequel ledit virus est un entérovirus, tel qu'un rhinovirus, un aphtovirus ou un virus de la poliomyélite.

15. Procédé selon une quelconque des revendications 1-12, dans lequel ledit virus est un virus de l'herpès, tel que le virus de l'herpès simplex, le virus de la pseudo-rage ou le virus de l'herpès bovin.

16. Procédé selon une quelconque des revendications 1-12, dans lequel ledit virus est un orthomyxovirus, tel que le virus de la grippe, un paramyxovirus, tel que le virus de la maladie de Newcastle, le virus respiratoire syncytial, le virus des oreillons ou un virus de la rougeole.

17. Procédé selon une quelconque des revendications 1-12, dans lequel ledit virus est un rétrovirus, tel que le virus de l'immunodéficience humaine ou dans lequel ledit virus est un parvovirus ou un papovavirus.

18. Procédé selon une quelconque des revendications 1-12, dans lequel ledit virus est un rotavirus ou un coronavirus, tel qu'un virus de la gastro-entérite transmissible ou un flavivirus, tel que le virus de l'encéphalite de la taïga ou le virus de la fièvre jaune.

19. Procédé selon une quelconque des revendications 1-12, dans lequel ledit virus est un togavirus, tel que le virus de la rubéole ou le virus de l'encéphalite équine du Vénézuéla, de l'Est ou de l'Ouest.

20. Procédé selon une quelconque des revendications 1-12, dans lequel ledit virus est un virus provoquant l'hépatite, tel que le virus de l'hépatite A ou le virus de l'hépatite B.

21. Procédé selon une quelconque des revendications 1-12, dans lequel ledit virus est un pestivirus, tel que le virus du choléra du porc ou un rhabdovirus, tel que le virus de la rage.

22. Procédé selon une quelconque des revendications 1-12, dans lequel ledit virus est un virus Bunyamwera, tel qu'un virus Hantaan.

23. Utilisation d'une cellule humaine immortalisée ayant une séquence codant au moins une protéine E1 d'un adénovirus ou son dérivé, homologue ou fragment fonctionnel dans son génome, laquelle cellule ne produit pas de protéines adénovirales structurelles pour la production d'un virus non-adénoviral pour une utilisation dans un vaccin, dans lequel ladite cellule humaine est dérivée d'une cellule embryonnaire de la rétine humaine.

24. Utilisation selon la revendication 23, dans laquelle ladite cellule humaine est une cellule telle que déposée à l'ECACC sous le numéro 96 022 940 ou un dérivé de celle-ci.

25. Utilisation selon la revendication 23-24, dans laquelle ladite cellule humaine comprend de plus une séquence codant E2A ou son dérivé ou analogue ou fragment fonctionnel dans son génome.

26. Utilisation selon la revendication 25, dans laquelle ledit E2A est sensible à la température.

27. Cellule humaine ayant une séquence codant au moins une protéine E1 d'un adénovirus ou de son dérivé, homologue ou fragment fonctionnel dans son génome, laquelle cellule ne produit pas de protéines adénovirales structurelles et ayant un acide nucléique codant un virus non-adénoviral, dans lequel ladite cellule humaine est dérivée d'une cellule embryonnaire de la rétine humaine.

28. Cellule humaine selon la revendication 27 qui est dérivée d'une cellule telle que déposée à l'ECACC sous le numéro 96 022 940.

29. Cellule humaine selon la revendication 27-28, qui comprend de plus une séquence codant E2A ou son dérivé ou analogue ou fragment fonctionnel dans son génome.

30. Cellule humaine selon la revendication 29, dans laquelle ledit E2A est sensible à la température.
